# FASCICULE DE BREVET EUROPEEN

(11) **EP 0 827 533 B1**
(45) Date de publication et mention de la délivrance du brevet: **14.06.2006**
(21) Numéro de dépôt: 96917526.4
(22) Date de dépôt: 21.05.1996
(51) Int. Cl.: C12N 9/24, C12P 19/14, C12S 3/02, C12S 3/04, C12S 3/08, C12S 3/12, C12S 9/00, C12S 11/00, C12Q 1/04, C12G 1/02, C12N 1/20

(54) **ENZYME ET MICROORGANISME DEGRADANT LE RHAMNOGALACTURONANE**
ENZYM UND MIKROORGANISMUS ZUM ABBAU VON RHAMNOGALAKTURONAN-II
ENZYME AND MICROORGANISM DEGRADING RHAMNOGALACTURONANE II

(30) Priorité: 23.05.1995 FR 9506142
(43) Date de publication de la demande: 11.03.1998
(73) Titulaire: INSTITUT NATIONAL DE LA RECHERCHE AGRONOMIQUE, 75341 Paris Cédex 07 (FR)
(72) Inventeur: PELLERIN, Patrice, F-34000 Montpellier (FR); BRILLOUET, Jean-Marc, F-34000 Montpellier (FR); DOCO, Thierry, F-34680 Saint-Georges-d'Orques (FR); WILLIAMS, Pascale, F-34090 Montpellier (FR); VIDAL, Stéphane, F-34000 Montpellier (FR); MOUTOUNET, Michel, F-34080 Montpellier (FR)
(74) Mandataire: Phélip, Bruno
(86) Numéro de dépôt international: PCT/FR1996/000758
(87) Numéro de publication internationale: WO 1996/037604

(56) Documents cités:
- EP-A- 0 225 496
- EP-A- 0 422 618
- EP-A- 0 570 075
- WO-A-92/19728
- WO-A-94/20612
- DE-A- 4 221 753
- DOMSCH K. H. ET GAMS W.: "Compendium of soil fungi" 1980 , ACADEMIC PRESS LTD , LONDON XP002002127 cité dans la demande voir page 597 - page 598
- DOMSCH K.H. ET GAMS W.: "Compendium of soil fungi" 1980 , ACADEMIC PRESS LTD , LONDON XP002002128 cité dans la demande voir page 560
- PLANT PHYSIOL., vol. 80, no. 4, 1986, pages 1012-1019, XP000600428 STEVENSON ET AL.: "Structure of plant cell walls : XVIII. An analysis of the extracellular poysaccharides of suspension-cultured sycamore cells"
- CARBOHYDRATE RES., vol. 218, 1991, pages 211-222, XP002012467 PUVANESARAJAH ET AL.: "Structural characterization of two oligosaccharide fragments formed by the selective cleavage of rhamnogalacturonan II : evidence for the anomeric configuration and attachment sites of apiose and 3-deoxy-2-heptulosaric acid" cité dans la demande
- METHODS ENZYMOLOGY, vol. 118, 1985, pages 3-40, XP000600280 YORK ET AL.: "Isolation and characterization of plant cell walls and cell wall components"
- CARBOHYDRATE RESEARCH, vol. 206, 1990, pages 105-115, XP000257186 SHOLS ET AL.: "Rhamnogalacturonase : a novel enzyme that degrades the hairy regions of pectins" cité dans la demande
- CARBOHYDRATE RESEARCH, vol. 206, 1990, pages 117-129, XP002002123 SCHOLS ET AL.: "Structural features of hairy regions of pectins isolated from apple juice produced by the liquefaction process" cité dans la demande
- BIOSCI. BIOTECH. BIOCHEM., vol. 56, no. 7, 1992, pages 1053-1057, XP002002124 SHINPEI MATSUHASHI ET AL.: "Simultaneous measurement of the galacturonate and neutral sugar contents of pectic substances by an enzymic-HPLC method"
- J. CELL. SCI., vol. 2, no. suppl, 1985, pages 203-217, XP002002125 DARVILL ET AL.: "Structure and function of plant cell wall polysaccharides"
- CARBOHYDRATE RESEARCH, vol. 243, 1993, pages 333-343, XP002002126 DOCO ET AL.: "Isolation and characterization of a rhamnogalacturonan II from red wine" cité dans la demande
- CARBOHYDRATE RESEARCH, vol. 261, 1994, AMSTERDAM NL, pages 335-342, XP002020190 HENK A. SCHOLS ET AL.: "The use of combined high-performance anion-exchange chromatography."

## Description

La présente invention de cette enzyme a pour objet une enzyme dégradant le rhamnogalacturonane II (RG-II) et ses dérivés, ainsi que son procédé d'obtention.

Elle a en outre pour objet un procédé d'obtention du RG-II.

Elle est également relative à l'utilisation de cette enzyme.

Le rhamnogalacturonane II est un constituant universel et très complexe de la paroi cellulaire des plantes (O'Neill et al., Methods in Plant Biochemistry (1990) 2:415-439). II appartient aux polysaccharides pectiques et est présent essentiellement au niveau de la lamelle moyenne et de la paroi primaire.

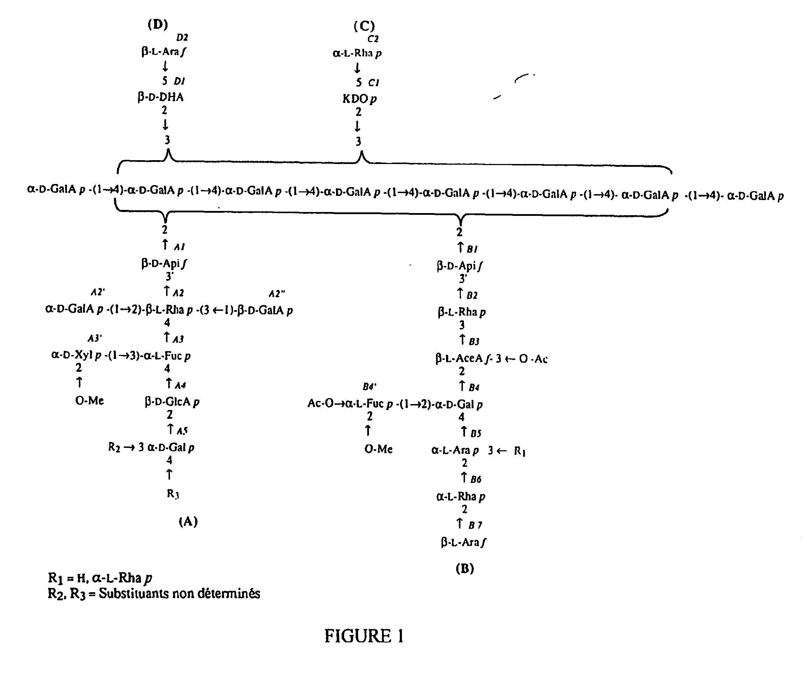

Sa structure est extrêmement complexe (figure 1) puisqu'il est composé de 12 monosaccharides différents pour un degré de polymérisation (dp) voisin de 30. Cette complexité est renforcée par la présence de sucres rares dans sa composition qui contient: de l'apiose ou 3-C-(hydroxyméthyl)-D-giycéro-tétrose; du KDO ou acide 2-céto-3-déoxy-D-manno-octulosonique; du DHA ou acide 3-déoxy-D-lyxo-2-heptulosarique, du 2-O-méthyl-fucose, du 2-O méthyl-xylose et enfin de l'acide acétique ou 3-C-carboxy-5-déoxy-L-xylose. Ce dernier monosaccharide constitue un marqueur spécifique du RG-II. Des monosaccharides plus habituels dans les polysaccharides pariétaux, le rhamnose, le fucose, l'arabinose, le galactose et les acides galacturonique et glucuronique, sont également présents dans la composition du RG-II mais le plus souvent avec des anoméries et des types de liaisons multiples et particuliers qui renforcent la spécificité et la complexité de la structure de la molécule.

L'organisation ultrastructurale du RG-II (figure 1) vient encore augmenter cette complexité (Puvanesarajah et al., Carbohydr. Res. (1991) 218:211-222). Une chaîne homologue et composée de 7 à 14 résidus d'acide galacturonique liés en α-(1→4) porte, en des positions non encore déterminées, 4 branches différentes: deux disaccharides et deux chaînes plus complexes octa- ou nonasaccharides. Des variabilités structurales sont observées pour la longueur de la chaîne homogalacturonique et pour l'extrémité de la branche B et semblent dues aux activités enzymatiques mises en oeuvre pour son obtention. D'autre part, la présence de substituants non encore identifiés a été détectée sur le résidu A5. En outre, la molécule de RG-II contient de 2 à 3 groupements méthyle qui estérifient les groupements carboxyliques des acides galacturoniques et deux groupements acétyle situés sur les résidus R3 (acide acérique) et B4' (2-O-méthyl-fucose).

Ainsi, la structure du RG-II a été généralement déterminée, même si la nature de certains substituants n'a pas encore été identifiée.

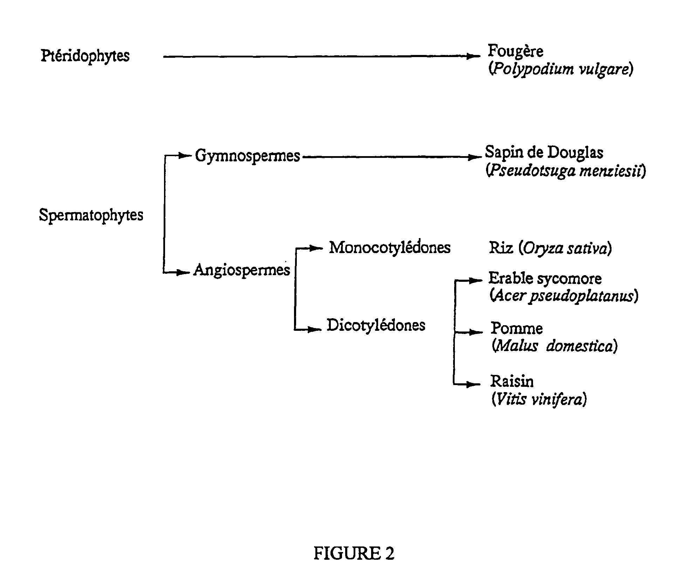

Dans la paroi cellulaire des plantes le RG-II est associé aux pectines natives mais le type exact de liaison entre protopectine et RG-II est inconnu à l'heure actuelle. De même, son rôle dans la paroi des végétaux est mal connu. On sait cependant qu'il est présent à travers tout le règne végétal (Albersheim et al., Biochem. Soc. Transactions (1994) 22:374-378); des ptéridophytes aux spermatophytes, des gymnospermes aux angiospermes, des monocotylédones aux dicotylédones (figure 2) et en quantité non négligeable dans la lamelle moyenne des parois. On sait aussi que sa structure est conservée dans les différentes plantes où il a été étudié (Albersheim et al., Biochem. Soc. Transactions (1994) 22:374-378).

De récents travaux ont montré qu'il pouvait agir comme polysaccharide signal et induire des réponses spécifiques de la plante (activité élicitrice) (Aldington et Fry,]. Exp. Botany (1994) 45:287-293). Cependant, son abondance, son ubiquité et la complexité de sa structure laissent supposer qu'il joue un rôle fondamental dans la cohésion des parois cellulaires des plantes et donc dans l'organisation cellulaire des tissus végétaux.

Le RG-II est libéré à partir des parois cellulaires sous l'action d'enzymes à activité pectolytique (pectine estérase, endo- ou exo-polygalacturonase) qui dégradent les chaînes lisses des pectines natives et libèrent le RG-II sous forme non dégradée (Darvill et al., Plant. Physiol. (1978) 62:418-422), comme l'illustre la figure 1. La variabilité de la longueur de la chaîne homogalacturonique rend compte des dégradations enzymatiques qui ont permis sa libération à partir des pectines natives (Whitcombe et al., Carbohydr.Res (1995) 271:15-29).

La complexité et l'originalité de la structure du RG-II le rendent particulièrement résistant à la dégradation par les enzymes présentes dans les extraits de plantes comme dans les préparations enzymatiques commerciales tant d'origine fongique que bactérienne.

Aucune activité de dégradation ne peut en effet être mise en évidence dans les préparations enzymatiques commerciales de dégradation des parois de plantes les plus riches en activités diverses. Les seules dégradations observées concernent les résidus arabinofuranose, rhamnopyranose ou galactopyranose situés à l'extrémité non-réductrice des chaînes latérales. Des exo-enzymes permettant la libération de tels résidus existent en effet couramment dans les préparations enzymatiques; c'est ce qui explique sans doute la variabilité observée entre les différentes préparations de RG-II rapportées selon les auteurs.

Lors du pressurage ou du broyage des fruits et des légumes, des activités pectolytiques sont libérées qui assurent la dégradation des chaînes homogalacturoniques des pectines natives . Ces activités sont renforcées par l'addition d'enzymes pectolytiques commerciales et par la flore de fermentation dans le cas des vins et des produits fermentés. Le RG-II est libéré sous forme intacte et en quantité souvent importante dans les jus et nectars de fruits, de légumes et dérivés, en particulier dans les vins.

Dans les vins, le RG-II représente l'un des polysaccharides majeurs (Doco et Brillouet, Carbohydr. Res. (1993) 243:333-343), sa concentration pouvant atteindre 100 mg/l. Il participe à de nombreux phénomènes indésirables, tels que le colmatage des membranes de filtration (Belleville et al., Enol. Vitic. Sci.(1991) 46:100-107), la formation de complexes instables avec d'autres macromolécules colloïdales et l'induction de phénomènes de précipitation. Son élimination passe obligatoirement par l'utilisation d'enzymes spécifiques de sa dégradation.

Plusieurs méthodes d'obtention du RG-II à partir de vin ont été décrites à ce jour:
- par hydrolyse enzymatique à l'aide d'endopolygalacturonases fongiques de parois cellulaires isolées à partir de cultures de cellules végétales en suspension (Darvill et al. Plant Physiol. (1978) 62:418-422),
- à partir d'une préparation enzymatique commerciale d'*Aspergillus niger,* le pectinol AC (Stevenson et al., Carbohydr. Res.(1988) 179:269-288); le RG-II y est présent à faible concentration et sa purification implique de nombreuses étapes d'élimination des protéines et de la matière colorante,
- à partir du vin (Doco et Brillouet, Carbohydr. Res. (1993) 243:333-343), la purification décrite a mis en oeuvre 4 étapes successives de chromatographie d'exclusion stérique et d'échange d'ions.

On notera que le rhamnogalacturonane I ou RG-1, bien qu'ayant un nom très proche est un constituant des parois cellulaires végétales totalement différent du RG-II tant au point de vue structural (sa structure est basée sur une alternance de rhamnose et d'acide galacturonique) que de sa dégradation par les enzymes. Des activités rhamnogalacturonase (Schols et al., Carbohydr. Res. (1990) 206:105-115) ou protopectinase (Sakamoto et Sakai, Carbohydr. Res. (1994) 259:77-91) ont déjà été décrites.

Il ressort de l'analyse ci-dessus de l'état de la technique que l'homme du métier ne connaissait aucune enzyme ou préparation enzymatique présentant une activité satisfaisante de dégradation du RG-II. Pourtant la présence de ce polysaccharide est une source de phénomènes indésirables tels que le colmatage des membranes de filtration, la formation de complexes instables avec d'autres macromolécules colloïdales et l'induction de phénomènes de précipitation.

Du fait de l'importance des activités économiques mises en cause, il était nécessaire de trouver une solution à ce problème.

Le demandeur a montré qu'il était possible d'isoler les activités enzymatiques responsables de la dégradation du RG-II et de ses dérivés à partir de microorganismes.

II a d'autre part mis au point un nouveau procédé d'obtention du RG-II permettant de l'obtenir en grandes quantités à partir de diverses sources végétales.

La présente invention a pour objet une préparatoin enzymatique caractérisée en ce qu'elle dégrade le RG-II et ses dérivés.

Pour la présente invention, on entend par RG-II, tout polysaccharide comprenant la structure indiquée à la figure 1, qu'il soit sous forme de monomère ou de dimère et toute molécule résultant de sa dégradation partielle et comportant au moins un fragment de chaîne A, B, C ou D caractéristique tant du point de vue composition que de sa séquence.

Dans ce qui suit toute mention relative au RG-II est susceptible de s'appliquer à tout dérivé de cette molécule.

Une telle préparatoin enzymatique présente avantageusement une activité de type endo-hydrolase.

Une telle préparatoin enzymatique peut en particulier présenter une activité endo-β-L-rhamnopyranosyl-(1→3')-D-apiofuranosyl hydrolase et endo-α-L-fucopyranosyl-(1→4)-L-rhamnopyranosyl hydrolase. Ces activités peuvent être identifiées par leur capacité à libérer la chaîne A, telle qu'elle apparaît sur la figure 1.

Une telle préparatoin enzymatique peut être produite par un microorganisme en particulier par un champignon du genre *Penicillium.*

De tels microorganismes présentant une activité de dégradation du RG-II et de ses dérivés, constituent un autre objet de la présente invention. Ils peuvent être en particulier les souches de *Penicillium* suivantes déposées le 19 Mai 1995 auprès de la Collection Nationale de Cultures des Microorganismes de l'Institut Pasteur (CNCM):
- souche de *Penicillium simplicissimum* déposée auprès de la CNCM sous le n°I-1577 (IPV1),
- souche de *Penicillium daleae* déposée auprès de la CNCM sous le n°I-1578 (LaV2).

Ces deux souches de champignons présentent un aspect caractéristique de champignons filamenteux cloisonnés avec formation rapide (dès le 6ème jour de culture) de spores couleur bleu-vert.

*P. daleae* est un champignon peu courant, toujours isolé à partir de sols forestiers et qui est connu pour sa capacité à dégrader l'amidon et les polysaccharides pectiques. Cette espèce a été décrite dans " Compendium of Soil Fungi, H.K. Domsch, W. Gams et T.H. Anderson (1980) Academic Press, London, page 560.

*P. simplicissimum* est plus commun et fréquemment isolé à partir de végétaux en décomposition. Cette espèce a été décrite dans " Compendium of Soil Fungi, H.K. Domsch, W. Gams et T.H. Anderson (1980) Academic Press, London, pages 597-598".

La présente invention est aussi relative à des préparations enzymatiques contenant une enzyme dégradant le RG-II et ses dérivés telle que décrite ci-dessus.

Ainsi, de telles enzymes peuvent être mises sous la forme de préparations contenant d'autres enzymes présentant d'autres activités.

L'enrichissement de telles préparations avec des activités permettant la dégradation du RG-II offre un potentiel entièrement nouveau pour toutes les applications requérant la dégradation partielle ou totale des parois cellulaires et des polysaccharides des plantes.

Des enzymes permettant la dégradation spécifique du RG-II et de ses dérivés peuvent être utilisés pour la dégradation ou la modification du RG-II ou de ses dérivés, et en particulier dans les applications suivantes:
- élimination du RG-II des jus de fruits, de légumes et de leurs dérivés, afin d'améliorer la filtrabilité, de faciliter la production de jus et de bases aromatiques concentrées, de favoriser les phénomènes de clarification et d'assurer une bonne stabilité des produits finis,
- nettoyage des membranes de micro- et ultra-filtration utilisées pour la clarification des jus de fruits, de légumes et de leurs dérivés,
- obtention de préparations de type macérase, c'est-à-dire permettant la dissociation des tissus cellulaires des végétaux jeunes avec une dégradation minimale des structures pariétales (production de nectars de fruits, de purées, de gelées et de concentrés de fruits et de légumes),
- obtention de préparations de types liquéfiantes, c'est-à-dire devant assurer l'hydrolyse complète des polysaccharides des parois cellulaires de plantes (production de jus et de bases aromatiques de fruits, de légumes et de boissons fermentées, de bière),
- production de pectines et d'aliments pour animaux à partir de résidus de végétaux (pulpes de betterave, résidus solides après pressurage des fruits...),
- production de cellulose à partir de végétaux; l'hydrolyse enzymatique des autres constituants polysaccharidiques permet en effet d'améliorer les rendements de production (industrie textile, production de papier).

Les préparations enzymatiques commerciales permettant la dégradation des parois cellulaires de plantes se doivent d'avoir des modes d'action biochimiques les plus précis et les mieux définis selon le type d'utilisation recherché. L'apport spécifique d'enzymes de dégradation du RG-II et ayant des modes d'action précis et bien déterminés va dans le sens d'une meilleure exploitation des potentiels technologiques des enzymes fongiques.

Les préparations enzymatiques selon l'invention décrites ci-dessus peuvent être préférentiellement obtenues par un procédé comprenant les étapes suivantes:
- mise en culture des microorganismes présentant une activité de dégradation du RG-II, ou de ses dérivés, dans un milieu de culture adapté à la production de ces enzymes.
- récupération de la préparation enzymatique dans le surnageant du broyat des microorganismes.

Préférentiellement un tel procédé comprend les étapes suivantes:
- mise en culture de microorganismes présentant une activité de dégradation du RG-II dans un milieu de culture adapté à ces microorganismes et contenant du RG-II,
- récupération des microorganismes,
- broyage des microorganismes,
- élimination du matériel insoluble, en particulier par filtration ou par centrifugation des microorganismes broyés, et
- récupération du surnageant contenant la préparation enzymatique.

Avantageusement, ce procédé est mis en oeuvre à l'aide des souches de *Penicillium* déposées auprès de la CNCM sous les N°I-1577 et N°I-1578.

De telles enzymes peuvent aussi être obtenues par génie génétique, après clonage du ou des gènes responsables de leur synthèse, ou par toute autre technique à la portée de l'homme du métier, en particulier par synthèse à partir d'acides aminés isolés, après identification de leur séquence.

Les procédés objets de la présente invention, en particulier les procédés de préparation et d'isolement des enzymes selon la présente invention, peuvent être mis en oeuvre par l'homme du métier à la seule lecture de la présente description et de ses connaissances générales. Néanmoins, il pourra se référer au manuel suivant : Methods in Microbiology, volumes 1 à 6, J.R. Morris et D.W. Ribbons (1969-1972), Academic Press, London, New York.

La présente invention est illustrée sans pour autant être limitée par les exemples qui suivent .

Les figures auxquelles font référence la présente description et ses exemples sont les suivantes.
La figure 1 représente de manière schématique la structure du rhamnogalacturonane II, comprenant ses quatre chaînes latérales A à D. Sur cette figure, R¹ représente un hydrogène ou un α -L-rhamnopyranose et R² et R3 représentent un hydrogène ou un autre substituant.
La figure 2 illustre la répartition de RG-II dans le règne végétal;
La figure 3 illustre le fractionnement des polysaccharides, sucres acides et sucres neutres, d'un vin sur une colonne de DEAE (Macroprep).
Les figures 4A et 4B sont des profils en chromatographie d'exclusion stérique à haute performance de deux fractions de RG-II isolées à partir d'un vin rouge;
La figure 5 montre un profil en chromatographie d'exclusion stérique haute performance sur colonne de Superdex-75 HR de deux fractions de RG-II purifiées à partir de concentré de vin: a: fraction III, dimère de RG-II (masse moléculaire de 9500 Da, élution à 18.5 min); b: fraction II, monomère de RG-II (masse moléculaire de 4750 Da, élution à 20,5 min);
La figure 6 est un schéma de purification du RG-II à l'échelle pilote;
La figure 7 est une comparaison des profils, déterminés en CES-HP sur colonne de Superdex-75 HR, des polysaccharides totaux d'un vin rouge (a) avec la fraction non retenue sur résine Relite® DIAION® (b) et retenue puis éluée à l'éthanol 20% (c);
La figure 8 illustre la dégradation de RG-II par un extrait cellulaire soluble de *Penicillium simolicissimum* (I-1577). Les spectres a, b et c correspondent respectivement au RG-II natif, et au RG-II après 24 heures et 48 heures de dégradation;
La figure 9 illustre la dégradation de RG-II par un extrait cellulaire soluble de la souche *P. daleae* LaV₂ (1-1578) (a:RG-II natif; b:après 96 heures; c: après 168 heures; d: après 190 heures).
La figure 10 illustre la dégradation, suivie par chromatographie CES-HP, de RG-II par une culture de *Penlcillium simplicissimum* (1-1577). Les courbes a à f représentent respectivement le spectre du RG-II natif, et les spectres du RG-II après 96 heures, 132 heures, 168 heures, 192 et 400 heures de croissance du champignon;
La figure 11 illustre la dégradation de RG-II par une culture de *P.daleae* (I-1578), suivie par chromatographie CES-HP, à différents temps (a: RGII natif; b: 72h; c: 120 h; d: 240h; e:264h; f: 336h; g:384h).
La figure 12 représente la structure de la figure 1 simplifiée. Les sites de coupure potentielle de l'enzyme selon la présente invention sont indiqués (flèches 1 et 2);
La figure 13 représente la structure de la fraction résiduelle du RG-II après sa dégradation par *Penicillium simplicissimum* et *Penicillium daleae.*
La figure 14 illustre la dégradation de RG-II dimérique par une culture de *P. daleae* (I-1578). Les spectres a à c représentent respectivement le spectre du RG-II dimérique natif et du RG-II après 168 et 300 heures de dégradation:
La figure 15 illustre la dégradation, suivie par chromatographie CES-HP, du RG-II purifié par un extrait cellulaire soluble total de *P.simplicissimum* (I-1577). Les spectres a à d correspondent respectivement au RG-II initial, et au RG-II après 72 heures, 120 heures et 148 heures d'incubation.
La figure 16 est une comparaison des profils en polysaccharides, déterminés en CES-HP, d'un vin rouge (spectre a) et après 48 heures d'incubation en présence de l'extrait enzymatique de IPV1 (spectre b).
La figure 17 est une comparaison en CES-HP des profils en polysaccharides d'un vin rouge (spectre a), et après 48 heures d'incubation en présence de Pectinex® Ultra Sp L seul (spectre b) ou associé à l'extrait enzymatique de la souche IPVI (spectre c).
La figure 18 est une comparaison en CES-HP des profils en polysaccharides d'un jus de pomme, obtenu par liquéfaction totale des fruits par la préparation enzymatique Rapidase® Liq) après 48 h d'incubation en l'absence (spectre a) et en présence (spectre b) de l'extrait enzymatique de la souche IPV1.
Les figures 19A et 19B illustrent la dégradation de tissus de betterave (figure 19A) par un extrait enzymatique de la souche IPV1, par comparaison au témoin (figure 19B).
Les figures 19C, 20A et 20C représentent respectivement les dégradations par des extraits enzymatiques de la souche IPV1 de tissus de carotte, de pomme, et de pomme de terre par comparaison aux témoins respectifs (figures 19D, 20B et 20D).

### EXEMPLE 1: PURIFICATION DU RHAMNOGALACTURONANE II PAR CHROMATOGRAPHIE D'ECHANGE D'ANIONS ET CHROMATOGRAPHIE DE TAMISAGE MOLECULAIRE, A PARTIR DE VIN.

Le RG-II est présent sous forme non dégradée notamment dans les jus de fruits, de légumes et leurs dérivés, en particulier fermentés. Le RG-II peut être purifié à homogénéité à partir de tous produits dérivés des plantes, en réalisant les étapes de purification suivantes:
1. Obtention des macromolécules en une étape soit par précipitation à l'éthanol 80%, soit par ultrafiltration.
2. Chromatographie préparative d'échange d'anions à pH acide.
3. Chromatographie de tamisage moléculaire (cette dernière étape qui permet d'arriver à un taux de pureté élevé peut être négligée si un taux de pureté de 80% de la préparation en RG-II est recherché).

### 1- Echantillon de vin et obtention des colloïdes:

Le vin utilisé a été obtenu à partir de Carignan noir récolté à maturité en Septembre 1991 au Domaine Expérimental de Pech-Rouge/Narbonne. 600 litres ont été concentrés sur membrane d'ultrafiltration Carbo Sep M5 (Tech Sep, France) à un seuil de coupure de 10.000. Les colloïdes totaux du vin concentré (volume final 25L) ont alors été précipités par addition de 4 volumes d'éthanol acidifié à 60mM HCl, lavés successivement à l'éthanol 80 et 90%, repris dans l'eau et dialysés contre un tampon citrate de sodium 40mM à pH 4,6. Le poids sec des colloïdes totaux (déterminé après dessalage et lyophilisation d'une fraction aliquote) représentait 296 g soit 0,5 g environ par litre de vin.

### 2. Chromatographie d'échange d'anions:

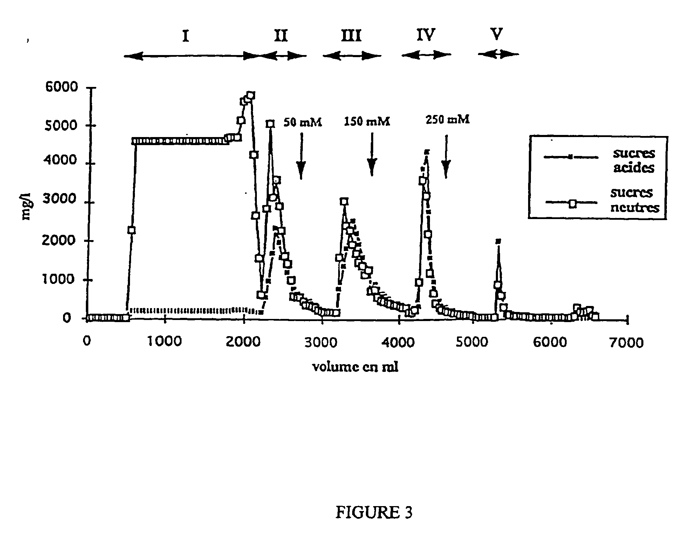

La solution de colloïdes à pH 4,6 a été fractionnée par chromatographie d'échange d'anions par 10 passages successifs sur une colonne de (5 x 80 cm) de DEAE-Macroprep (BioRad, USA) équilibrée à 20,5 ml/min dans un tampon citrate de sodium 40 mM à pH 4,6. Les polysaccharides neutres ou faiblement chargés ont été élués dans le tampon d'injection (fraction 1). Les fractions contenant le RG-II ont été éluées d'abord par passage de la concentration en tampon citrate à 50 mM (fraction II) puis par addition de 50 (fraction III) et 150 mM (fraction IV) de NaCl au tampon d'élution (figure 3). Ces trois fractions représentaient respectivement, 7,9%, 6,6% et 4,1 % des colloïdes totaux, exprimés en poids sec.

### 3- Purification du RG-II à homogénéité par chromatographie d'exclusion stérique:

Le RG-II présent dans les fractions II et III a été purifié à homogénéité par chromatographie d'exclusion stérique sur une colonne (5 x 75 cm) de Sephacryl S-400 HR équilibrée à 7 ml/min dans un tampon acétate de sodium 50 mM à pH 5 contenant 50 mM de NaCl.

Les fractions contenant le RG-II ont été finalement dialysées contre de l'eau avant lyophilisation.

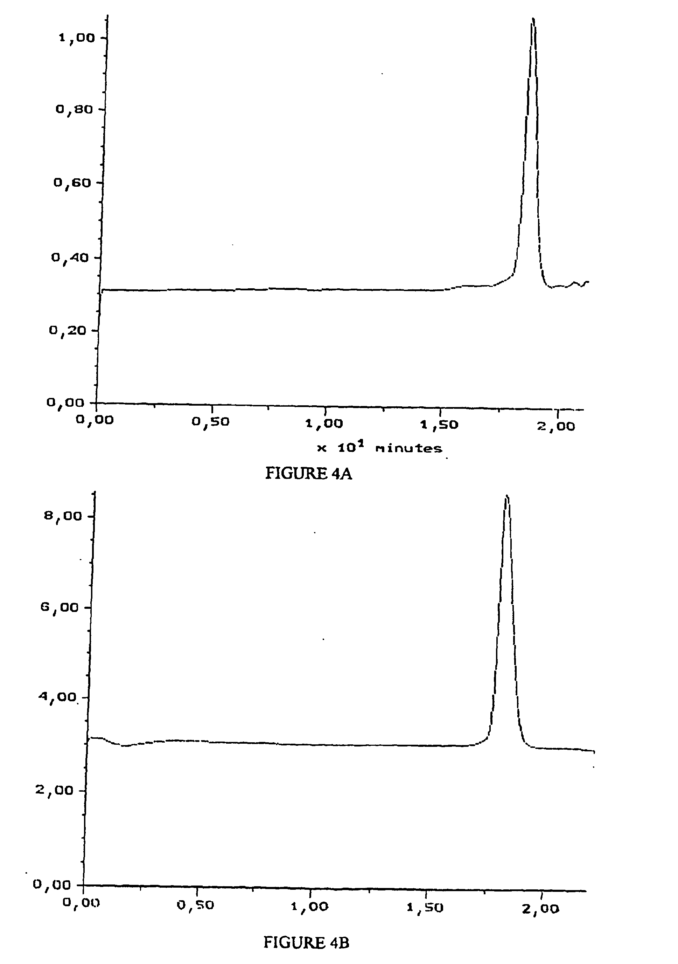

Les deux échantillons de RG-II obtenus présentaient un profil parfaitement homogène en chromatographie d'exclusion stérique à haute performance (CES-HP, analyse sur deux colonnes Shodex OHPak KB-803 et KB-805 en série, éluant LiN03 0,1 M à 1 ml/min, détection réfractométrique) (figure 4) et représentaient, respectivement 4,4 et 4,6 % des colloïdes totaux de l'échantillon de vin. Le taux de RG-II total de l'échantillon de vin utilisé était supérieur à 50 mg/l puisqu'il faut y ajouter le RG-II contenu dans la fraction IV qui n'a pas été purifié à homogénéité.

### 4- Composition des fractions de RG-II purifiées à partir du vin:

Les deux fractions de RG-II purifiées présentent toutes deux la composition caractéristique du RG-II (tableau 1). Elles ne différent pas de manière significative au vu de l'analyse de leur composition.

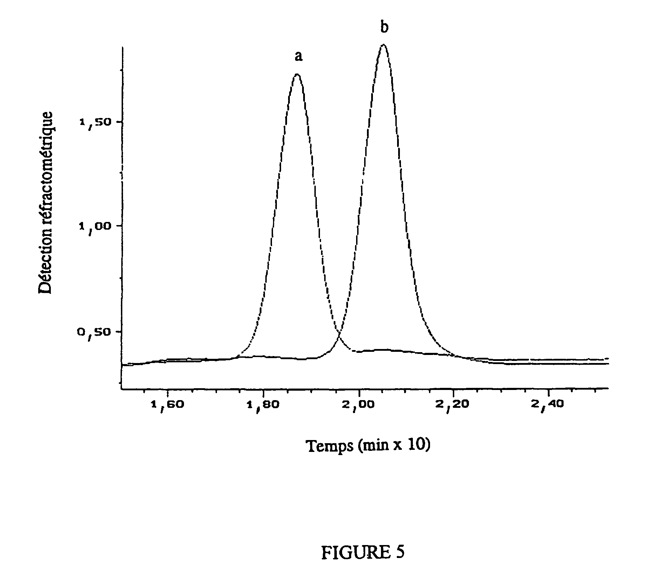

Les fractions II et III obtenues sur DEAE-Macroprep ont été analysées sur colonne de Superdex-75HR (1 x 30 cm, exclusion à 14 min), un support de tamisage moléculaire. Cette analyse (figure 5) révèle que la fraction II comprend majoritairement des monomères de RG-II (élution à 20,5 min, poids moléculaire 4750 Da) tandis que la fraction III contient plus de 95 % de dimère de RG-II (élution à 18;5 min, poids moléculaire 9500 Da).

La préparation de RG-II de la fraction II a été utilisée par la suite pour le travail de criblage et d'induction d'activités enzymatiques de dégradations spécifiques.

### EXEMPLE 2: Obtention de RGII par chromatographie d'adsorption à partir de vinasses.

Les vinasses utilisées ont été obtenues par distillation sous vide et concentration de 300 hl de vin rouge (mélange de vins de plusieurs cépages):
Les vinasses obtenues par distillation ont été concentrées par évaporation sous vide sur un système à flots tombants. Les sels de bitartrate sont éliminés après décantation dans ce concentrat puis les vinasses sont à nouveau concentrées sur le même système. Au total le vin a été concentré 30 fois et les colloïdes totaux des 1000 litres de vinasse concentrée obtenus ont été précipités par addition de 4 volumes d'éthanol 90%. Le précipité est repris dans 300 litres d'eau pour constituer la solution de vinasses utilisée comme source de RG-II.

### Chromatographie d'adsorption

Le suivi des séparations chromatographiques est assuré par CLHP sur colonnes Shodex (voir exemple 1 ou sur colonne Superdex-HR 75 (Pharmacia; débit 0,6 ml/min) couplée à un système de détection réfractométrique.

### (a) Sur charbon actif

Le charbon actif possède une forte capacité d'adsorption vis-à-vis de nombreuses molécules. Dans la présente invention, le charbon actif a été retenu pour sa capacité à séparer le RG-II des différentes classes de polysaccharides pectiques. Tous les polysaccharides sont en effet adsorbés sur le charbon actif mais le RG-II est désorbé à des concentrations en alcool inférieures à 40%. Deux types de charbon ont été testés.

### Charbon actif pulvérulent

Un litre de solution de vinasses diluée au cinquième a été mis en contact avec 100 grammes de charbon actif en poudre (Norit® SA⁺) pendant 30 minutes. Puis, la solution a été filtrée sur fritté afin d'éliminer les particules de charbon. Le charbon retenu sur le filtre est remis en suspension dans 1 litre d'éthanol à 40 % et le mélange est agité régulièrement pendant 30 minutes puis filtré sous vide. Le RG-II élué est alors analysé par CLHP. L' analyse de composition de cette fraction (tableau 2) indique un degré de pureté voisin de 50% pour le RG-II.

### - Charbon actif extrudé:

Ce type de charbon nécessite un temps de contact beaucoup plus long (48 heures) avec la solution de vinasses pour adsorber le RG-II mais présente l'avantage de faciliter les étapes de filtration. D'autre part, la masse de charbon nécessaire est le double de celle de charbon pulvérulent.

Un litre de solution de vinasses diluée au cinquième a été mise en contact avec 200 grammes de charbon actif extrudé (Norit® RO-08 Supra) pendant 48 heures. Après élimination de la fraction non adsorbée, la fraction contenant le RG-II a été désorbée par mise en solution dans l'éthanol 40% pendant 24 heures. Le RG-II obtenu présente le même degré de pureté que dans le cas de l'utilisation du charbon pulvérulent.

### (b) Sur résine mixte de polystyrène-divinylbenzène (Résine Relite® DIAION® SP411).

La résine Relite® DIAION® SP411 est une résine synthétique non polaire constituée de copolymères de polystyrène-divinylbenzène. Elle retient le RG-II aux dépens des autres polysaccharides présents dans les extraits végétaux. Ces derniers seront donc séparés en 2 fractions par chromatographie d'adsorption sur résine Relite® DIAION® SP411: une fraction non retenue renfermant les polysaccharides différents du RG-II et une fraction retenue contenant le RG-II.

### - Purification de RG-II à l'échelle pilote

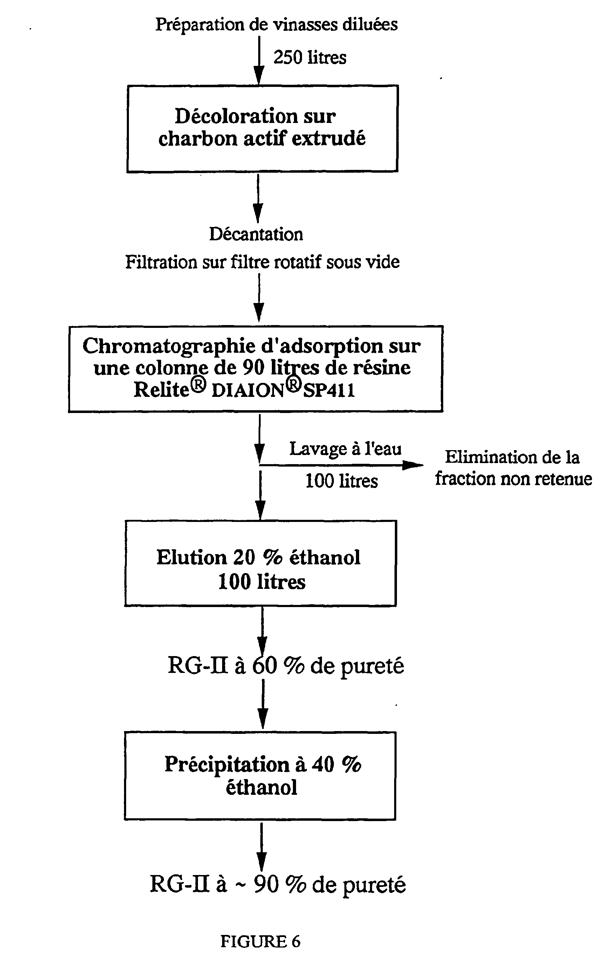

Le schéma de purification de RG-II à l'échelle pilote à partir des vinasses est représenté sur la figure 6. 250 litres de solution de vinasse diluée au cinquième (2,5 volumes de colonne) ont été décolorés rapidement sur charbon actif Norit® RO-08 Supra puis passés sur une colonne de 90 litres de Relite® DIAION® SP411 à un débit de 40 I/h (temps de contact de 2 heures pour une adsorption du RG-II sur ce support chromatographique).La colonne a été lavée par 100 litres d'eau. La fraction non retenue est donc constituée du volume de l'injection et du lavage. L'élution du RG-II a été obtenue par passage de 100 litres d'éthanol à 20% suivi d'un lavage de la colonne par 200 litres d'eau.

Le RG-II ainsi obtenu (1 kg pour la préparation totale de vinasses) présente un degré de pureté voisin de 60% (tableau 2). Le RG-II a été précipité par addition d'éthanol (concentration finale 40 % d'éthanol). Le précipité ainsi obtenu contient du RG-II à 90% de pureté.

### EXEMPLE 3: OBTENTION DE RG-II, PAR CHROMATOGRAPHIE D'ADSORPTION, A PARTIR DE VINS

Du vin (vin rouge cépage Merlot 94, vin blanc Chardonnay 94) a été désalcoolisé et concentré 2 fois par évaporation sous vide, sur évaporateur rotatif.

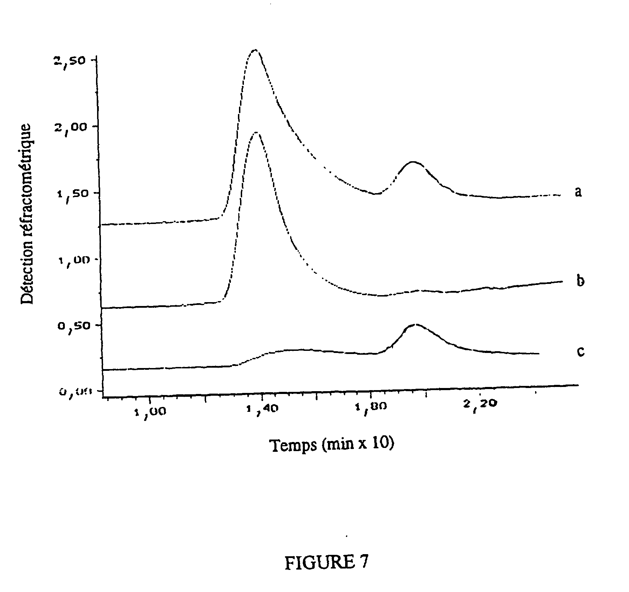

Des fractions de 15 ml sont chargées sur 50 ml de Relite® DIAION® SP411 à 25 ml/h. Puis, la colonne est lavée par 50 ml d'eau. Le RG-II est alors élué avec 50 ml d'éthanol 20% et la colonne est lavée par 100 ml d'eau. L'analyse CLHP (figure 7) montre que le RG-II est adsorbé quantitativement sur la résine puisqu'il est absent dans la fraction non retenue sur résine et qu'il est récupéré quantitativement par élution avec l'éthanol 20%. Le degré de pureté de la solution de RG-II est proche de 50% pour le vin rouge (Tableau 2) et de 35% pour le vin blanc.

### EXEMPLE 4: OBTENTION DE RG-II A PARTIR DE MOUT DE RAISIN.

L'Amberlite® XAD 2 est une résine copolymère de polystyrène-divinylbenzène non polaire. 100 ml de moût de raisin blanc obtenu par broyage de baies de cépage Grenache ont été injectés sur une colonne de 100 ml de résine XAD2 équilibrée dans l'eau. Le RG-II a été élué par un volume de méthanol 60% avec un degré de pureté de 40% environ. Les résultats de l'analyse de l'éluat figurent dans le tableau 2.

### EXEMPLE 5 - OBTENTION DE RG-II A PARTIR DE JUS DE FRUITS ET DE LEGUMES.

Des extraits solubles ont été obtenus à partir de 0,6 kg de pommes, de tomates et de carottes pelées et coupées en dés dans 200 ml d'acide ascorbique (concentration finale 3mM). Les jus de fruits et de légumes utilisés ont été obtenus par liquéfaction enzymatique par actions combinées de préparations enzymatiques commerciales liquéfiantes (Pectinex® Ultra SPL; Novo Ferment et Rapidase® Liq; Gist-Brocades) pendant 24 heures à 45°C. La réaction de liquéfaction est stoppée par dénaturation des enzymes sous l'effet de la chaleur. Les résidus solides ont été éliminés par centrifugation et le surnageant a été utilisé comme source de RG-II.

### Purification de RG-II de fruits et de légumes après liquéfaction enzymatique.

Les jus de fruits et légumes obtenus par liquéfaction enzymatique sont filtrés sur filtre papier avant d'être passés sur une colonne (2,5 x 30 cm) équilibrée en eau contenant 50 ml de Relite® DIAION® SP411 à 25 ml/h. La colonne est lavée par 50 ml d'eau avant élution du RG-II par 50 ml d'éthanol 20% suivi d'un lavage de la colonne par 100 ml d'eau.

Les préparations de RG-II ainsi obtenues présentent des degrés de pureté supérieurs à 80%).

Les résultats de leur analyse figurent dans le tableau 2.

### EXEMPLE 6: CRIBLAGE DE MICROORGANISMES DEGRADANT LE RG-II SOUS FORME MONOMERIQUE.

Le RG-II est un constituant universel des parois cellulaires de plantes, sa dégradation dans la biosphère est assurée par les microorganismes des écosystèmes naturels: sols, composts, boues de stations d'épuration...

A partir d'échantillons naturels, ont été isolées deux souches de champignons appartenant à deux espèces de *Penicillium* qui utilisent le RG-II comme seule source de carbone et d'énergie.

### 1- Milieu de culture :

Le milieu de culture utilisé pour le criblage de microorganismes utilisant le RG-II comme source de carbone et d'énergie a été réalisé comme indiqué dans le tableau 3. Il s'agit d'un milieu de culture minéral auquel est ajoutée une solution de RG-II monomérique à une concentration finale de 2 à 10 mg/ml. Les cultures contenant des champignons ont été débarrassées des bactéries contaminantes par addition de trois antibiotiques dans le milieu: pénicilline G, streptomycine et tétracycline.

### 2- Criblage de microorganismes utilisant le RG-II comme source de carbone et d'énergie.

Des échantillons ont été prélevés à partir d'écosystèmes naturels variés (sols agricoles ou forestiers, tourbes, composts, boues de stations d'épuration, fruits en décomposition...) et mis en culture à 25 et 37°C sur le milieu de culture minéral ajusté à 10 mg/ml en RG-II monomérique. Chaque fois que des croissances de microorganismes ont été observées, les cultures ont été repiquées plusieurs fois sur le même milieu. Cette procédure a permis d'isoler, dans un premier temps, plusieurs cultures assurant leur croissance sur le milieu RG-II. La dégradation du RG-II dans le milieu a été contrôlée parallèlement par chromatographie d'exclusion stérique à haute performance et seules ont été retenues les cultures où était observée une telle dégradation en parallèle avec la croissance de microorganismes.

Les microorganismes assurant la dégradation du RG-II ont été isolés après étalement des cultures en boîtes de Pétri sur milieu gélosé obtenu par addition d'agarose à 2% dans le milieu de culture (Tableau 3) et contenant 2,5 mg/ml de RG-II monomérique. Après une semaine d'incubation à 25°C, des clones ont été prélevés et inoculés en milieu de culture liquide afin de contrôler leur capacité à dégrader le RG-II.

### 3- Choix des souches dégradant le RG-II:

Deux souches de champignons filamenteux ont finalement été retenues car elles présentaient les propriétés requises:
1- Croissance rapide et parfaitement corrélée avec la diminution du RG-II dans le milieu.
2- La disparition du RG-II en quantité était observée en parallèle avec un décalage de son volume d'élution en HP-SEC ce qui indiquait la présence d'activités enzymatiques de dégradation de type endo-hydrolase.
3- Le niveau final de dégradation du RG-II atteignait 70% après une semaine de culture à 25°C.

Ces deux cultures numérotées E et K ont été utilisées par la suite pour la production d'enzymes de dégradation du RG-II et l'étude des modes d'actions enzymatiques. Elles sont cultivées sans agitation au contact de l'air à 25°C en milieu de culture liquide contenant 5 mg/ml de RG-II, en présence de trois antibiotiques: pénicilline G, streptomycine et tétracycline.

### EXEMPLE 7: PRODUCTION D'ENZYMES DE DEGRADATION DU RG-II:

Les différentes espèces de *Penicillium* sont connues pour leur capacité à produire et à relarguer dans les milieux de culture des enzymes de dégradation de polysaccharides. La capacité des deux souches isolées à produire des enzymes de dégradation du RG-II a été vérifiée.

### 1- Recherche d'enzymes de dégradation du RG-II dans le surnageant de culture:

Les souches *P. daleae* LaV2 (N° de dépôt CNCM I-1578) et *P. simplicissimum* IPV1 (n° de dépôt CNCM I-1577) ont été mises en culture à 25°C sur milieu contenant 2,5 mg/ml de RG-II monomérique. Après 96 h de culture, 1 ml de milieu de culture contenant du mycélium en croissance a été prélevé, filtré stérilement sur filtre 0,22 µm puis additionné de 2 mg/ml de RG-II natif. Le milieu contenant les enzymes de dégradation et le RG-II est mis à incuber à 25°C et des fractions de 25 µl sont prélevées aux temps O,20 et 45 h et analysées en CES-HP. La comparaison entre les profils d'élution du RG-II aux différents temps d'incubation fait clairement ressortir l'absence d'activités de dégradation du RG-II dans le milieu de culture. Ces activités ont donc été recherchées dans le cytoplasme et le périplasme des champignons.

### 2- Production d'enzymes de dégradation du RG-II dans les extraits cellulaires totaux des champignons:

Les souches précitées *P. daleae* LaV2 et *P. simplicissimum* IPV1 ont été mises en culture à 25°C sur 4 ml de milieu contenant 6 mg/ml de RG-II monomérique. Après 140 h de culture, le mycélium a été récupéré par centrifugation avant broyage (aux billes de verre) à 4°C pendant 4 min dans un tampon MES (acide morpholino-éthane-sulfonique)/KOH 50 mM ajusté à pH 6, additionné de 1 mM de PMSF (phényl méthyl sulfonyl fluorure) et 1 mM de DTT (dithiothréitol). Le surnageant cellulaire a été récupéré par centrifugation à 10.000 g x 5 min.

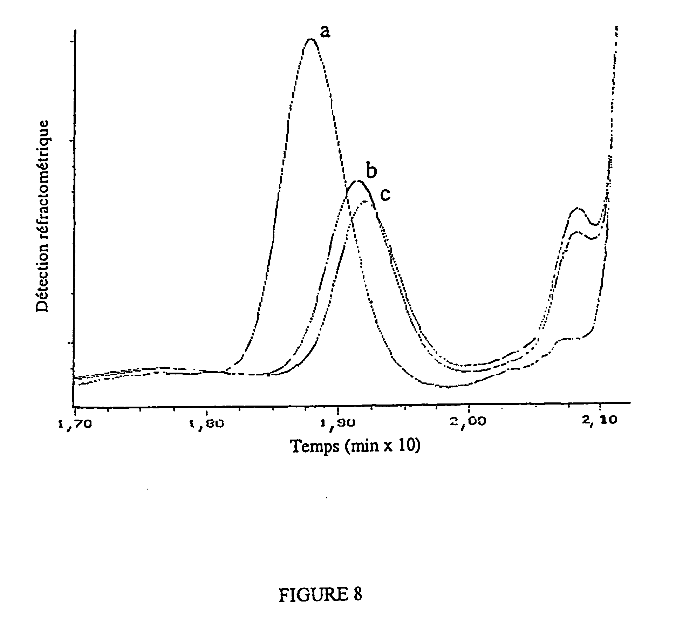

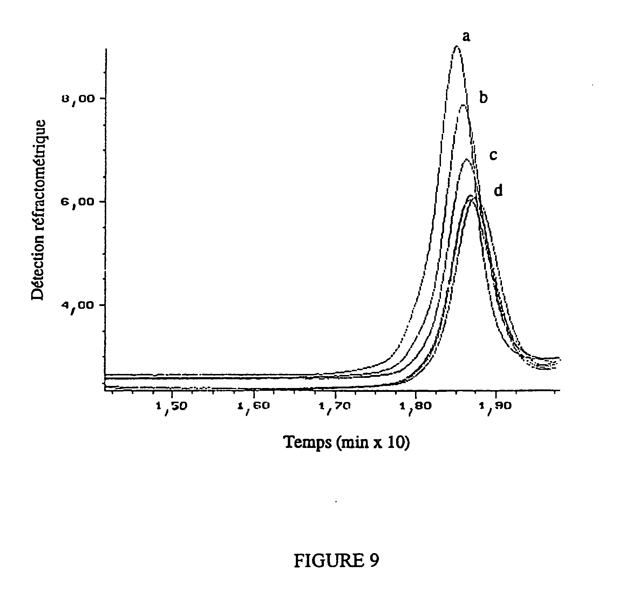

La présence d'enzymes de dégradation du RG-II dans l'extrait cellulaire soluble a été testée par addition de RG-II natif à 2,5 mg/ml au surnageant après broyage et centrifugation, incubation à 25°C et suivi par CES-HP de la dégradation du RG-II. L'analyse des profils aux temps 24 et 48 h en comparaison avec le RG-II natif (figures 8 et 9) montre une forte dégradation (50%) du RG-II dès 24 h qui se poursuit jusque 48 h pour donner 40 % de molécule résiduelle.

Les activités enzymatiques assurant la dégradation du RG-II sont donc présentes dans l'extrait cellulaire soluble et peuvent donc être obtenues après broyage des champignons. Les deux souches de *Penicillium* isolées sont donc bien productrices d'enzymes de dégradation du rhamnogalacturonane II.

### EXEMPLE 8 CARACTERISATION DES ACTIVITES DE DEGRADATION DU RG-II.

Les deux espèces de *Penicillium* isolées et identifiées produisent des enzymes qui dégradent le RG-II dans le milieu de culture. Le mode d'action de ces enzymes a été étudié dans un premier temps en suivant la dégradation du polysaccharide dans le milieu au cours de la croissance des champignons. Cette approche présente l'inconvénient de ne permettre de suivre que la fraction du RG-II non assimilée par le champignon, elle permet cependant de rendre compte des activités mises en oeuvre et de leur mode d'action.

### 1- Réalisation d'une cinétique de dégradation du RG-II par Penicillium simplicissimum:

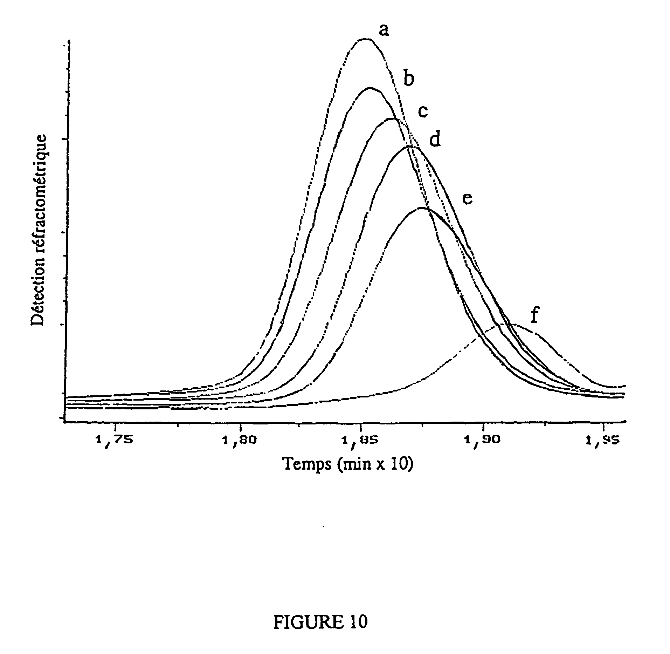

La souche de *P. simplicissimum* IPV1 a été mise en culture en Erlenmeyer à 25°C sur 10 ml de milieu à 5 mg/ml de RG-II monomérique. Des fractions de 1 ml ont été prélevées aux temps O, 96, 132, 168 et 192 h. Le pH du milieu a été alors réajusté à 5 par addition de 20 µl de HCl 1 M. Un nouveau prélèvement a été effectué à 360 h, la culture étant finalement arrêtée après 400 h d'incubation. Chaque prélèvement a été analysé en CES-HP (figure 10) ce qui permet de suivre la dégradation au cours de la croissance des champignons. Les différentes fractions prélevées ont été dessalées sur une colonne (1 x 50 cm) de Bio-Gel P-6 équilibrée dans un tampon acétate de sodium 100 mM à pH 4.

Pour chaque temps d'incubation, une analyse structurale complète de la fraction de RG-II en cours de dégradation a été réalisée. Cette analyse comprend:
- La quantification du taux de dégradation du RG-II.
- La détermination de la composition en oses neutres et en acides uroniques.
- La détermination des types de liaisons entre les résidus constituant la molécule.
- La détermination de la longueur de la chaîne homo-galacturonique.

L'ensemble de ces analyses permet de donner l'état de la molécule de RG-II pour chaque prélèvement et donc de suivre sa dégradation au cours du temps. Ces résultats rendent compte du mode de dégradation enzymatique de la molécule au cours du temps.

### 2- Réalisation d'une cinétique de dégradation du RG-II par Penicillium daleae.

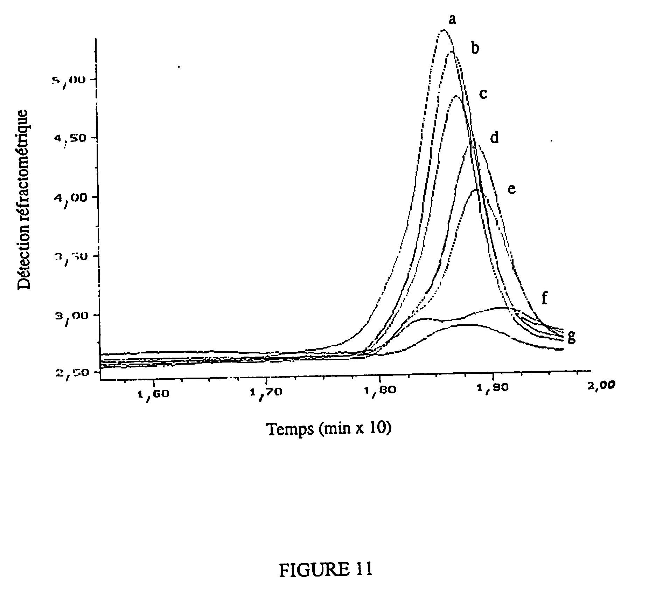

Pour le suivi de la dégradation de RG-II par *P.daleae* LaV2, 500 mg de préparation de RG-II monomérique ont été préalablement saponifiés (2 h à 4°C dans NaOH 50 mM) puis réduits (6 h à 4°C en présence de 2,5 g de NaBH4) afin de marquer l'extrémité réductrice de la molécule. La souche de *P. daleae* LaV2 a été mise en culture en Erlenmeyer à 25°C sur 6 ml de milieu à 5 mg/ml de RG-II saponifié et réduit. Des fractions de 0,6 ml ont été prélevées aux temps 0, 72, 120, 168, 240, 264, 336 et 384 h. Chaque prélèvement a été analysé en CES-HP (figure 11) afin de suivre la dégradation au cours de la croissance des champignons. Les différentes fractions prélevées ont été déssalées sur une colonne (1 x 30 cm) de Superdex-75 HR équilibrée à 0,6 ml/min dans un tampon formiate d'ammonium 30 mM à pH 5,2.

Pour chaque temps d'incubation, une analyse structurale complète de la fraction de RG-II en cours de dégradation a été réalisée. Cette analyse comprend:
- la quantification du taux de dégradation du RG-II
- la détermination de la composition en oses neutres et en acides uroniques sous forme de dérivés triméthylsilylés après méthanolyse.
- la détermination des types de liaisons entre les résidus constituant la molécule par analyse de méthylation incluant la réduction des acides uroniques au lithium triéthylborodeutéride (Pellerin et al. 1995 Carbohydr. Res. 277: 135-143).
- la détermination de la longueur de la chaîne homo-galacturonique.

L'ensemble de ces analyses permet de donner l'état de la molécule de RG-II pour chaque prélèvement et donc de suivre sa dégradation au cours du temps. Ces résultats rendent compte du mode de dégradation enzymatique de la molécule au cours du temps.

### 3- Mécanisme de la dégradation du RG-II:

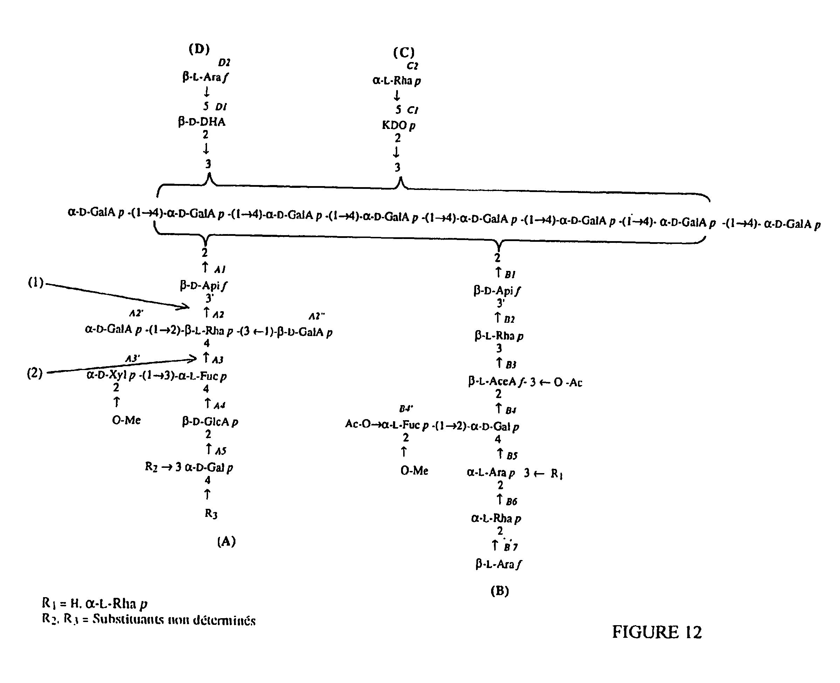

L'analyse de la composition (tableaux 4 et 5) et des types de liaison pour chaque résidu présent dans la molécule (tableaux 6 et 7) permet de suivre l'état de la molécule résiduelle du RG-II au cours du temps et fait ressortir que le RG-II est dégradé , pour les deux souches de *Penicillium,* par une série d'enzymes qui agissent de manière séquentielle:
a) La première étape de dégradation consiste en la rupture des liaisons entre le résidu de β-L-rhamnose trisubstitué et les résidus de α-L-fucose et de β-D-apiose ce qui conduit à la perte de la chaîne A (figure 12) qui est assimilée par le champignon. Cette étape se produit au cours des premiers jours de culture, parallèlement à un léger raccourcissement de la chaîne homo-galacturonique qui passe de 8-9 à 7 résidus en moyenne et à la perte partielle des résidus terminaux d'arabinofuranose (B7 et D2) et de rhamnopyranose (C2).
b) L'élimination complète des résidus A2 à A5 de la chaîne A portée par le résidu de β-D-apiose semble lever la résistance de la molécule de RG-II aux dégradations enzymatiques puisqu'une deuxième phase de dégradation est alors observée qui se caractérise par:
   - Un fort raccourcissement de la chaîne homo-galacturonique qui passe à une taille moyenne de 4 résidus d'acide galacturonique,
   - la perte du résidu A1.
   - La chaîne B toujours liée à la chaîne homo-galacturonique par l'intermédiaire du β-D-apiose, subit des modifications concernant son extrémité terminale non réductrice, à savoir la perte quantitative du résidu d'arabinofuranose terminal et la perte des résidus de α-L-rhamnose (résidus B6 et B7).
   - Les résidus de Kdo et de Dha semblent affectés partiellement par la dégradation enzymatique.

Toutes les dégradations observées au cours de cette deuxième phase peuvent être obtenues à l'aide d'enzymes connues : β-D-apiosidase, β-L-arabinosidase, α-L-rhamnosidase, endo- ou exo-polygalacturonase.

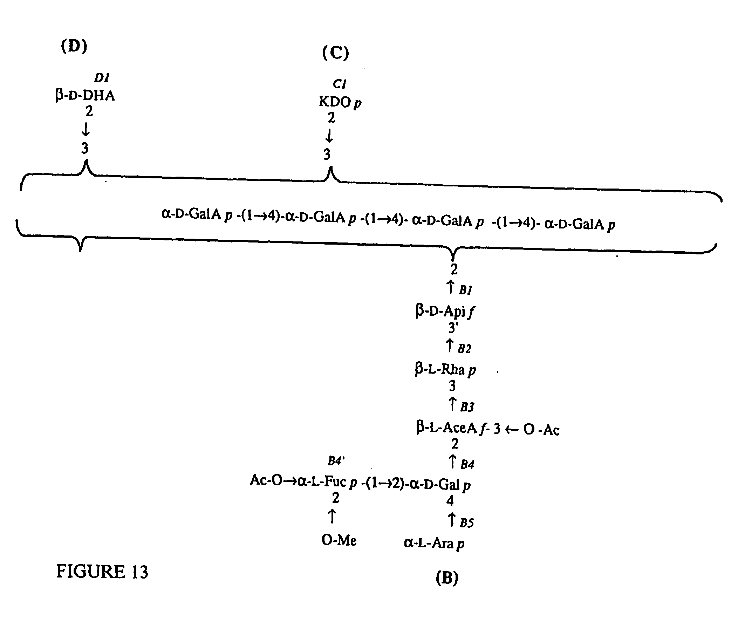

La molécule résiduelle à la fin de la croissance des champignons correspond quantitativement à la chaîne B (résidus B₁ à B₅), aux résidus de Kdo (C1) et de Dha (D1) portés par un oligosaccharide acide de degré de polymérisation moyen de 4 et représente 20 à 30 % de la molécule initiale de RG-II (figure 13).

La première étape qui met en oeuvre deux activités enzymatiques de type endo-est donc l'étape dé qui permet la dégradation de la molécule de RG-II. La libération quantitative de la chaîne A rend en effet la molécule sensible à l'effet d'enzymes aux modes d'action connus et qui sont fréquemment rencontrées dans les préparations enzymatiques commerciales.

La production d'enzymes à activité de type endo-β-L-rhamnopyranosyl-(1→3')-D-apiofuranosyl hydrolase (1) et endo-α-L-fucopyranosyl-(1→4)-L-rhamnopyranosyl hydrolase (2) par *P. daleae* LaV2 et *P. simplicissimum* IPV1 est donc l'étape déterminante qui permet à ces champignons d'assurer leur croissance en utilisant le RG-II. C'est donc l'utilisation de ces enzymes à activité endo-β-L-rhamnopyranosyl-(1→3')-D-apiofuranosyl hydrolase ou endo-α-L-fucopyranosyl-(1→ 4)-L-rhamnopyranosyl hydrolase qui permet la dégradation enzymatique poussée du RG-II. La spécificité de l'activité (1) est forte puisque la chaîne B n'est pas affectée par son activité hydrolytique bien qu'elle soit reliée à la chaîne homo-galacturonique par l'intermédiaire d'une liaison rhamnosyl-apioside du même type. Cette différence de comportement de l'activité (1) vis-à-vis des chaînes A et B peut s'expliquer:
- Soit par la différence entre les substituants du rhamnose: une chaîne liée en C3 dans le cas de B, une chaîne liée en C4 et deux monosaccharides liés en C2 et C3 dans le cas de A.
- Soit par une localisation différente de ces deux chaînes au sein de la molécule de RG-II.

La présence dans une préparation enzymatique de l'une ou l'autre de ces deux activités permet la libération de la chaîne A du RG-II et lève donc le problème de la non-dégradabilité du RG-II par les enzymes habituelles des préparations pectinolytiques. En effet, les autres enzymes permettant une dégradation plus poussée du RG-II interviennent après le départ de la chaîne A sous l'action des enzymes précitées. Cette deuxième étape de dégradation fait intervenir des enzymes à activités:
- β-D-apiosidase
- β-L-arabinosidase
- α-L-rhamnosidase
- endo-polygalacturonase
- exo-polygalacturonase.

### EXEMPLE 9: OBTENTION D'UNE PREPARATION DE RG-II DIMERIQUE ET DEGRADATION PAR Penicillum daleae:

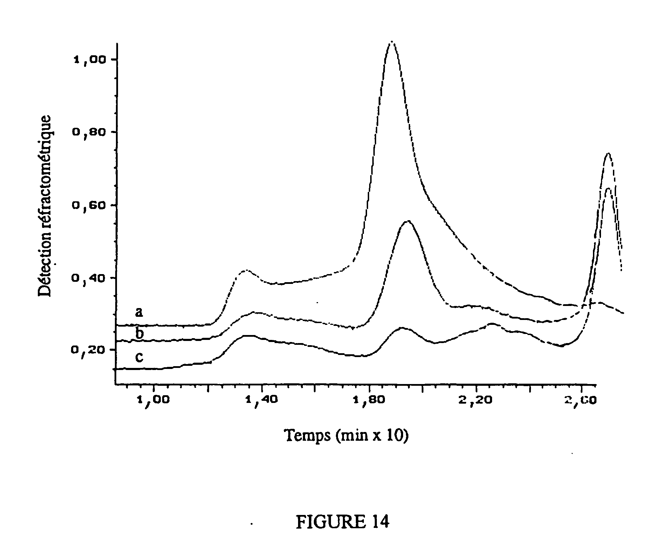

La souche de *P. daleae* a été mise en culture sur milieu.contenant 5 mg/ml de préparation de RG-II obtenue par passage de concentrés de vinasse sur résine Relite® DIAION® (exemple 2). Cette préparation contient 60 % environ de RG-II qui se trouve sous forme dimérique ainsi que le montre l'analyse sur colonne de Superdex-75 HR. Le suivi par CLHP (figure 14) du milieu de culture de champignon montre une forte dégradation du RG-II. Les contaminants de plus haut poids moléculaire (essentiellement des mannanes présents dans les concentrés de vinasse) ne sont pas dégradés.

Les souches de *Penicillium* isolées sur RG-II monomérique présentent donc la capacité à dégrader le RG-II sous forme de dimère. Les enzymes faisant l'objet de la présente demande de brevet sont donc actives sur les deux formes de la molécule.

### EXEMPLE 10: OBTENTION D'UNE PREPARATION ENZYMATIQUE DEGRADANT LE RG-II:

Une préparation enzymatique contenant les activités de dégradation du RG-II, en particulier les activités de type endo-β-L-rhamnopyranosyl-(1→ 3')-D-apiofuranosyl hydrolase ou endo-α-L-fucopyranosyl-(1→4)-L-rhamnopyranosyl hydrolase a été obtenue à partir d'une culture de *P. simplicissimum* IPV1 réalisée comme suit:

120 ml de milieu de culture, contenant 6 mg/ml de RG-II purifié ont été répartis en 8 boîtes de Pétri. La souche *P. simplicissimum* IPV1 a été mise en culture à 25°C pendant 6 jours. Le mycélium (1,2 g en poids frais) a été alors récupéré par centrifugation avant broyage comme décrit ci-dessus (exemple 3) dans le tampon MES/KOH 50 mM ajusté à pH 6, contenant 1 mM de PMSF et de DTT. L'extrait enzymatique total (6 ml) a été finalement obtenu par centrifugation et utilisé pour les exemples suivants.

### EXEMPLE 11: DEGRADATION DU RG-II PURIFIE PAR L'EXTRAIT ENZYMATIQUE DE P. simplicissimum IPV1:

Le mélange suivant a été mis à incuber à 25°C en présence de 0,02 % de NaN₃:
- 500 µl de tampon acétate de sodium 50 mM, pH 4,8;
- 12,5 µl d'une solution de RG-II purifié (tableau 1, fraction II) à 200 mg/ml,
- 25 µl d'extrait enzymatique total de *P. simplicissimum* IPV1 obtenu selon l'exemple 5.

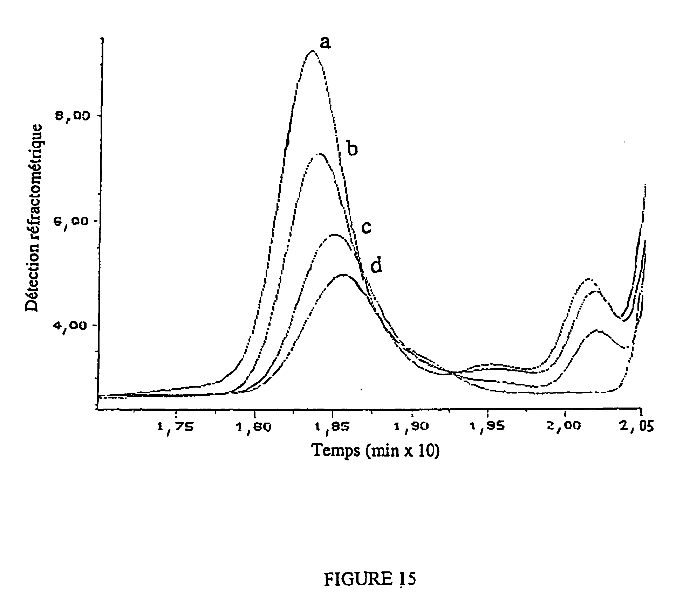

Des prélèvements de 25 µl ont été réalisés aux temps 0, 72, 120 et 148 h et analysés en CES-HP. La présence d'enzymes à activité de type endo-β-L-rhamnopyranosyl-(1→3')-D-apiofuranosyl hydrolase ou endo-α-L-fucopyranosyl-(1→4)-L-rhamnopyranosyl hydrolase est confirmée par la dégradation du RG-II (figure 15). Le profil de dégradation est d'ailleurs comparable à celui obtenu en réalisant un suivi dans le surnageant de culture de P. *simplicissimum* (figure 10). Des produits de dégradation de la molécule de RG-II apparaissent dans la zone de fractionnement de bas poids moléculaire (figure 15).

### EXEMPLE 12: DEGRADATION DES POLYSACCHARIDES D'UN VIN PAR L'EXTRAIT ENZYMATIQUE DE P. simplicissimum IPV1:

L'extrait enzymatique total contenant les enzymes de dégradation du RG-II, en particulier les activités de type endo-β-L-rhamnopyranosyl-(1 → 3')-D-apiofuranosyl hydrolase ou endo-α-L-fucopyranosyl-(1→ 4)-L-rhamnopyranosyl hydrolase a été testé pour sa capacité à dégrader le RG-II présent dans les vins.

Les polysaccharides totaux d'un vin rouge (cépage Carignan noir) ont été obtenus par ultrafiltration du vin (2 ml) sur membrane Centricon 30 (Amicon, USA). Le rétentat (100 µl) a été repris par 1,9 ml de tampon acétate 50 mM pH 4,8. Les essais suivants ont été mis à incuber à 25°C, en présence de 0,02 % de NaN₃:
a: 200 µl de rétentat contenant les polysaccharides totaux du vin 5 µl de H₂O
   2O µl tampon MES de broyage
b : 200 µl de rétentat contenant les polysaccharides totaux du vin
   5 µl de H₂O
   20 µl d'extrait enzymatique total de *P. simplicissimum* IPV1 obtenu selon l'exemple 9.
c : 200 µl de rétentat contenant les polysaccharides totaux du vin
   5 µl de Pectinex® Ultra Sp-L (Novo Ferment, Suisse)
   20 µl tampon MES de broyage.
d: 200 µl de rétentat contenant les polysaccharides totaux du vin
   5 µl de Pectinex® Ultra Sp-L
   20 µl d'extrait enzymatique total de *P. simplicissimum* IPV1 obtenu selon l'exemple 9.

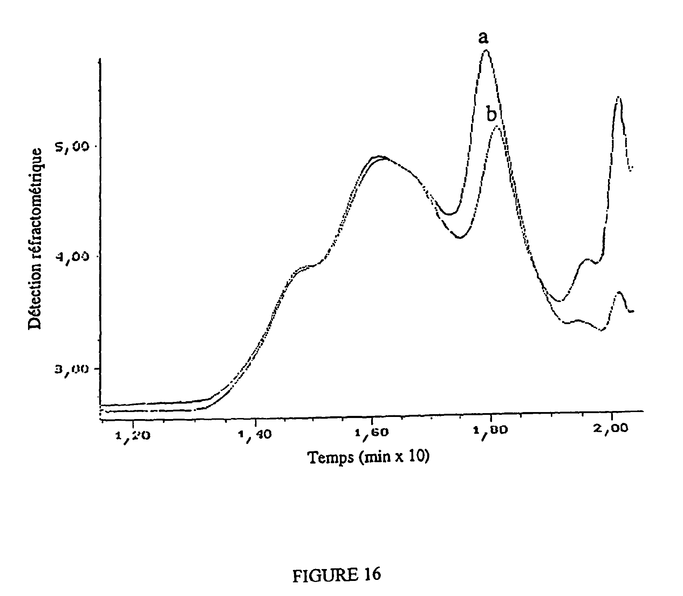

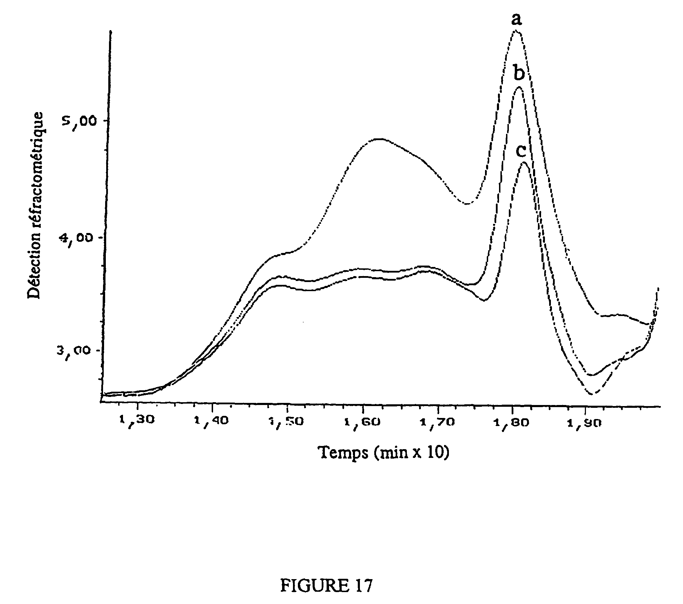

Après 48 h, 25 µl de chaque essai ont été prélevés et analysés en CES-HP. L'analyse des résultats (figures 16 et 17) fait ressortir :
- La dégradation du RG-II du vin (pic caractéristique élué à 18,2 min) par l'extrait enzymatique de *P. simplicissimum* IPV1 (figure 16).
- La dégradation des autres polysaccharides du vin (mannoprotéines, arabinanes et arabinogalactanes) par la préparation enzymatique commerciale Pectinex® Ultra Sp-L, tandis que le pic caractéristique de RG-II reste intact (figure 17).
- L'effet complémentaire des deux préparations enzymatiques est confirmé (figure 17) par la dégradation de l'ensemble des polysaccharides du vin dans l'essai c.

L'extrait enzymatique total de *P. simplicissimum* IPV1 contenant les enzymes de type endo-β-L-rhamnopyranosyl-(1→ 3')-D-apiofuranosyl hydrolase ou endo-α-L-fucopyranosyl-(1 →4)-L-rhamnopyranosyl hydrolase contient donc bien des activités enzymatiques absentes d'une préparation enzymatique commerciale produite à partir d'*Aspergillus aculeatus* et riche en activités cellulolytiques, hémicellulasiques et pectinolytiques, en particulier en activité rhamnogalacturonase (Schols et al., 1990, Carbohydr. Res, 206, 105-115).

L'addition des enzymes conformes à la présente invention en association avec d'autres activités pectinolytiques conduit à une dégradation plus poussée des polysaccharides d'un vin et permet donc l'amélioration de la filtrabilité et de la prévention de la formation des troubles colloïdaux et des précipités.

### EXEMPLE 13: DEGRADATION DES POLYSACCHARIDES D'UN JUS DE POMME PAR L'EXTRAIT ENZYMATIQUE DE P. simplicissimum IPV1:

Un jus de pomme a été réalisé à partir de 1 kg de pommes (variété Starking): les pommes entières ont été hachées en lamelles de 1 cm d'épaisseur, additionnées de 1 g d'acide ascorbique et de 500 µl de préparation enzymatique Rapidase® Liq (Gist Brocades, France) et laissées 2 h sous agitation à 50°C. Les fruits ont été liquéfiés sous l'action de la préparation enzymatique commerciale, le jus étant alors obtenu par centrifugation. La préparation de Rapidase® Liq contient des niveaux élevés d'activités de type pectinase (pectine, lyase, endo- et exo-polygalacturonase, rhamnogalacturonase,arabanase,...) hémicellulases (galactanases, xylanases...) et cellulases.

1,5 ml de jus de pomme clair a été ultrafiltré sur membrane Centricon 30 (Amicon, USA). Le rétentat (100 µl) a été repris par 1,4 ml de tampon acétate 50 mM pH 4,8 et les essais suivants ont été mis à incuber à 25°C, en présence de 0,02% de NaN₃:
a: 200 µl de rétentat contenant les polysaccharides totaux du jus de pomme 50 µl de H₂O
b: 200 µl de rétentat contenant les polysaccharides totaux du jus de pomme 50 µl d'extrait enzymatique total de *P. simplicissimum* IPV1.

Après 48 h, 25 µl de chaque essai ont été prélevés et analysés en CES-HP.

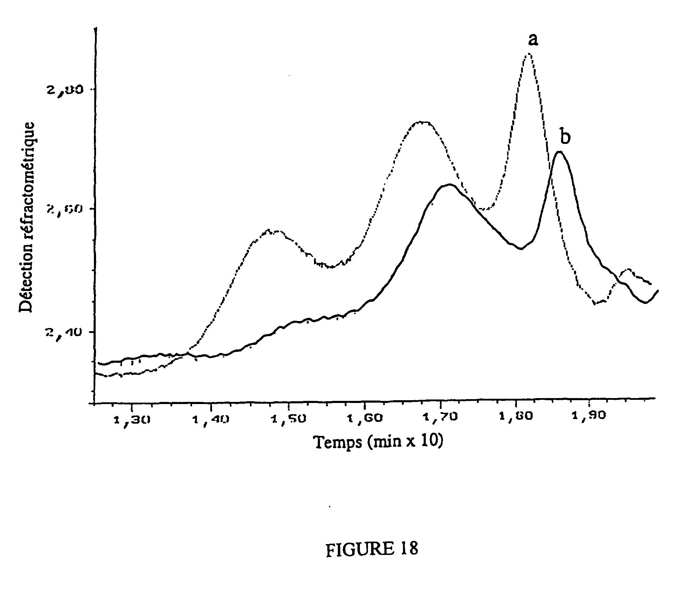

L'analyse des résultats (figure 18) fait ressortir l'effet complémentaire des enzymes de dégradation du RG-II puisque l'addition de l'extrait enzymatique de *P. simplicissimum* IPV1 permet une dégradation poussée de l'ensemble des polysaccharides résiduels après traitement des pommes par la préparation Rapidase® liq. En particulier, le pic caractéristique de RG-II (élué à 18,2 min) subit une forte dégradation. De plus, la dégradation des structures de plus haut poids moléculaire résistantes à l'action des enzymes présentes dans Rapidase® Liq, suggère que ces fractions contiennent également des fragments de RG-II.

L'extrait enzymatique total de *P. simplicissimum* IPV 1 dans lequel les activités de type endo-β-L-rhamnopyranosyl-(1→3')-D-apiofuranosyl hydrolase ou endo-α-L-fucopyranosyl-(1→4)-L-rhamnopyranosyl hydrolase ont été mises en évidence contient donc bien des activités enzymatiques absentes d'une préparation enzymatique commerciale produite à partir d'*Aspergillus niger* et décrite comme possédant l'ensemble des activités cellulolytiques, hémicellulasiques, pectinolytiques (y compris de type rhamnogalacturonase) connues. L'effet complémentaire et additionnel des enzymes de dégradation du RG-II est donc bien confirmé par cet essai.

### EXEMPLE 14: MACERATION DE FRUITS ET DE LEGUMES PAR L'EXTRAIT ENZYMATIQUE DE P. simplicissimum:

L'activité de dissociation des tissus végétaux de l'extrait enzymatique total de *P. simplicissimum* IPV1 a été vérifiée sur les fruits et légumes suivants:
- Betterave rouge
- Carotte
- Pomme de terre "Bintge"
- Pomme Golden.

Des fragments de 158 x 4 x 2 mm de chaque fruit ou légume ont été placés dans 2 ml de tampon acétate 50 mM pH 4,8 et additionnés:
- de 200 µl de tampon MES de broyage pour les témoins.
- de 200 µl d'extrait enzymatique total de *P. simplicissimum* IPV1 pour les essais enzymatiques.

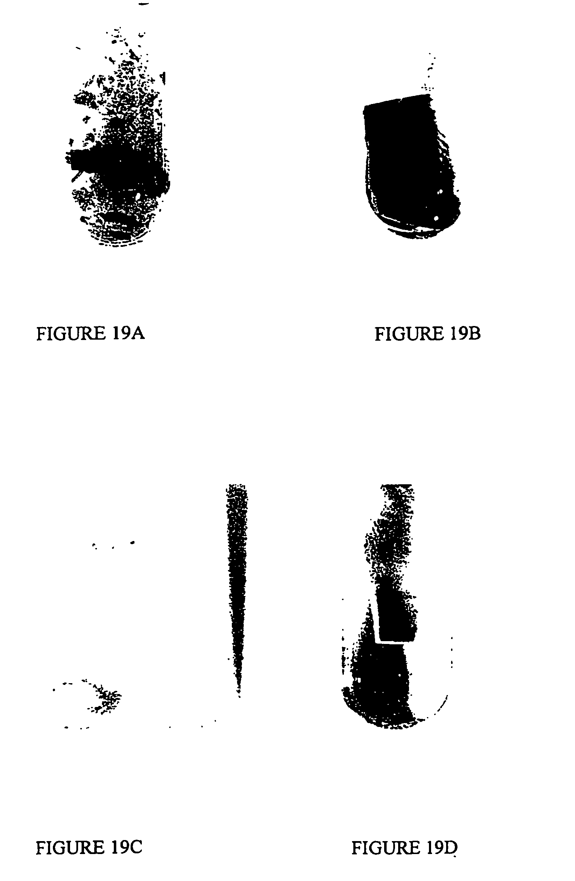

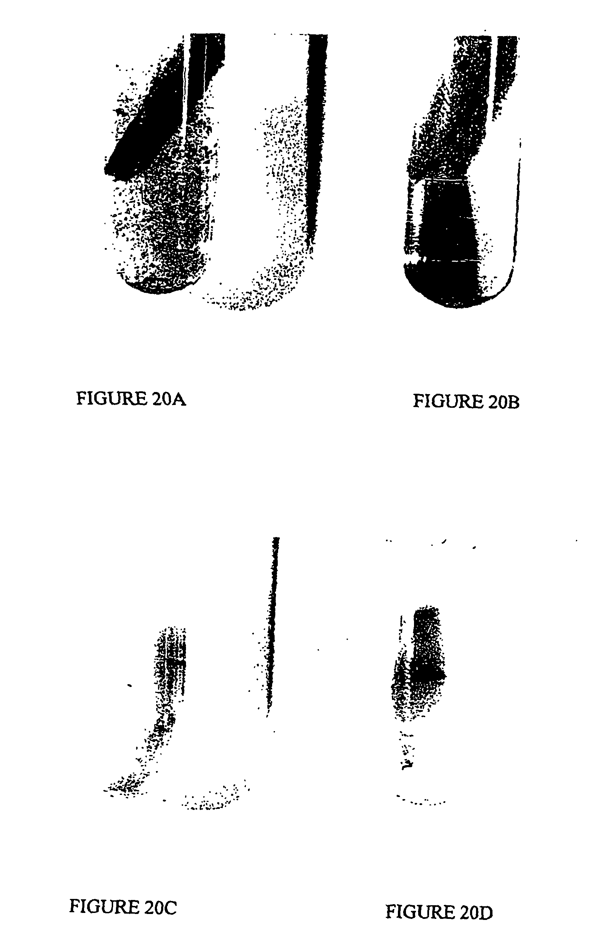

Les différents essais ont été mis à incuber à 25°C, en présence de 0,02 % de NaN₃, pendant 72 h avant d'être agités vigoureusement au vortex (2 x 10 sec) et photographiés. L'analyse des résultats fait ressortir:
- Une dilacération quasi totale des tissus pour la betterave et la carotte (figure 19).
- Un effet macérant accompagné de l'apparition de cellules en suspension pour la pomme de terre et la pomme (figure 20).

L'extrait enzymatique total de *P. simplicissimum* IPV 1 contenant les activités de type endo-β-L-rhamnopyranosyl-(1→3')-D-apiofuranosyl hydrolase ou endo- α̅-L-fucopyranosyl-(1→4)-L- rhamnopyranosyl hydrolase possède donc un effet de dissociation des tissus végétaux.

### CONCLUSION:

Les activités enzymatiques de dégradation du RG-II peuvent donc être utilisées seules ou en combinaison avec d'autres enzymes en phase liquide ou sur des supports solides et permettre:
- la dégradation à un degré élevé, par exemple 70%, du RG-II dans les jus de fruits, de légumes et de leurs dérivés, afin d'améliorer la filtrabilité, de faciliter la préparation de jus concentrés, de favoriser les phénomènes de clarification et d'assurer une bonne stabilité des produits finis.
- le nettoyage des supports de micro- et ultra-filtration utilisés pour la filtration de jus de fruits, de légumes et de leurs dérivés.

Ces activités enzymatiques favorisent la dégradation des parois cellulaires des plantes et sont donc utilisables seules ou en combinaison avec d'autres enzymes dans toutes les applications nécessitant la macération, la liquéfaction ou l'hydrolyse totale ou partielle de tissus végétaux, en particulier:
- dans les préparations de type macérase pour la production de nectars de fruits, de purées, de gelées et de concentrés de fruits, de légumes et de leurs dérivés, y compris le vin;
- dans les préparations liquéfiantes pour la production de jus et de bases aromatiques de fruits, de légumes et de leurs dérivés, y compris le vin, la production de bière.
- pour la production de pectines à partir de résidus de végétaux tels que pulpes de betterave, résidus solides après pressurage des fruits...
- pour la production d'aliments pour animaux à partir de matériel végétal.
- pour la production de cellulose à partir de végétaux pour l'industrie textile (en particulier à partir de coton), la production de papier.
- pour la production à partir de résidus de végétaux, d'oligosaccharides à activité élicitrice de réactions de défense des plantes et pouvant être utilisées comme produits phytosanitaires.

**TABLEAU 1 Composition de deux fractions de RG-II isolées à partir du vin**

| | Fraction II | Fraction III |
|---|---|---|
| Etat de la molécule | monomère | dimère |
| Proteines ^{a} | 0,6 | 0,6 |
| Acides Uroniques ^{a} | 36,9 | 39 |
| Sucres Neutres ^{a} | 27,3 | 24,9 |
| Methanol ^{a} | 1.4 | 1.3 |
| Acide Acétique ^{a} | 1,6 | 1,8 |
| Rhamnose ^{b} | 31,8 | 35,4 |
| 2-*O*-CH3-Fucose ^{b} | 6,3 | 6,5 |
| Fucose ^{b} | 3,7 | 5.2 |
| 2-*O*-CH3-Xylose ^{b} | 4,8 | 4,8 |
| Apiose ^{b} | 7,4 | 7,5 |
| Arabinose ^{b} | 25 | 23,7 |
| Galactose^{b} | 19,1 | 15.8 |
| Acide Acérique^{b} | 1,2 | 2,2 |
| Acide galacturonique ^{b} | 38,2 | 37,2 |
| Acide Glucuronique ^{b} | 3,3 | 3,4 |
| Kdo ^{b} | 4,4 | 5 |
| Dha ^{b} | 2,6 | 2,5 |

| | | |
|---|---|---|
| a % de matière sèche | | |
| b % molaire | | |

**TABLEAU 2 Composition (en pourcentage molaire) et degré de pureté des préparations de RG-II obtenues à partir de différents extraits végétaux et sur différents supports chromatographiques**

| Echantillons | Vin concentré | Pomme | Carotte | Tomate | Vin | Moût de raisin | Vinasses concentrées | |
|---|---|---|---|---|---|---|---|---|
| Supports utilisés | Ech. anions DEAE-Macroprep | Rélite Diaion SP411 | Rélite Diaion SP411 | Rélite Diaion SP411 | Rélite Diaion SP411 | Amberlite XAD 2 | Rélite Diaion SP411 | Charbon SA + |
| 2-O-Me-Fucose | 6,3 | 5,0 | 6,2 | 6,1 | 1,1 | 2,0 | 1,8 | 2,0 |
| Rhamnose | 31,8 | 29,9 | 31,2 | 20,8 | 13,6 | 22,3 | 14,6 | 22,5 |
| Fucose | 3,7 | 10,8 | 7,9 | 14,7 | 1,7 | 6,0 | 1,7 | 1.7 |
| 2-O-Me-Xylose | 4,8 | 3,9 | 4,9 | 4,7 | 0.9 | 3,8 | 1,5 | 1,6 |
| Arabinose | 25,0 | 20,1 | 23,7 | 20,9 | 52,3 | 22,6 | 29,3 | 13,0 |
| Xylose | - | - | - | - | 0,9 | 14,9 | 0,8 | 1,1 |
| Apiose | 7,3 | 7,2 | 2,5 | 8,0 | 1,5 | 9,1 | 2,4 | 2,9 |
| Mannose | 0.9 | 8,8 | 4,8 | 10,8 | 8,1 | 2,2 | 16,7 | 42,6 |
| Galactose | 19,1 | 12,0 | 17,2 | 14,2 | 15,0 | 3,0 | 27.7 | 11,8 |
| Glucose | 1,1 | 2,3 | 1,6 | - | 4,9 | | 3,4 | 0,9 |
| % de pureté | 97 | 95 | 96 | 82 | 45 | 40 | 57 | 52 |

**TABLEAU 3 Milieu de culture de champignons filamenteux:**

| - Milieu minéral: | |
|---|---|
| NH₄NO₃ | 2 g/l |
| K₂HPO₄ | 1 g/l |
| MgSO₄,7H₂O | 0,5 g/l |
| KCI | 0,5 g/l |
| Sulfate de fer | 10 mg/l |

| - Solution de Heller: | |
|---|---|
| ZnSO₄ | 1 g/l |
| MnSO₄,H₂O | 0,1 g/l |
| CuSO₄,5H₂O | 0,03 g/l |
| AlCl₂ | 0,03 g/l |
| NiCl₂,6H₂O | 0,03 g/l |
| KI | 0,01 g/l |
| Acide borique | 1 g/l |

| - Solution de vitamines: | |
|---|---|
| Vit B1 | 0,08 g/l |
| Vit H | 0,08 g/l |
| Réalisation du milieu de culture: | |
| - milieu minéral | 1 ml |
| - Solution de Heller | 1 µl |
| - Solution de vitamines | 1 µl |
| - Streptomycine (50 mg/ml) | 2 µl |
| - Tétracycline (5 mg/ml) | 2 µl |
| - Pénicilline (10 000 U/ml) | 10µl |
| - Polysaccharide | 5 mg/ml |
| Culture réalisée à température ambiante (25°C) à la lumière. | |

**Tableau 4 Composition (en % de la matière sèche initiale) du RG-II au cours d'une cinétique de dégradation par P. simplicissimum.**

| | | Initial | 96 h | 132 h | 168 h | 192 h | 400 h |
|---|---|---|---|---|---|---|---|
| Oses Neutres | | 28,2 | 28,5 | 23,1 | 19,1 | 18,5 | 10,3 |
| Acides Uroniques | | 40,4 | 35,3 | 25,9 | 18,1 | 16,4 | 9,9 |
| Oses Neutres+Ac. Uroniques | | 68,6 | 63,8 | 49,0 | 37,2 | 34,9 | 20,2 |
| Monosaccharides | Résidu n° | | | | | | |
| 2-O-Me-Fucose | B4' | 2,1 | 2,4 | 2,1 | 2,0 | 2,1 | 2,0 |
| Rhamnose | A2,B2,B6,D2 | 9,4 | 9,1 | 7.2 | 6,4 | 5,4 | 2,9 |
| Fucose | A3 | 1,1 | 1,1 | 0,6 | 0,1 | 0,1 | 0,0 |
| 2-O-Me-Xylose | A3' | 1,4 | 1,6 | 1,2 | 0,1 | 0,0 | 0,0 |
| Arabinose | B5,B7,C2 | 6,2 | 6,0 | 5,5 | 5,0 | 4,5 | 1,5 |
| Apiose | A1,B1 | 2,2 | 2,7 | 2,5 | 2,4 | 2,2 | 1,4 |
| Galactose | A5,B4 | 5,5 | 5,0 | 3,5 | 2,7 | 2,4 | 2,5 |
| Ac. galacturonique | A2',A2", poly-GalA | 23,3 | 24,2 | 19,5 | 18,9 | 15,2 | 8,5 |
| Ac. Glucuronique | A4 | 3,2 | 3,0 | 1,8 | 0,3 | 0,2 | 0,0 |
| dp moyen de la chaîne homogalacturonique | | 8 à 9 | 7 à 8 | 7 | 6 à 7 | 6 | 4 |
| % Molécule résiduelle | | 100 | 93 | 71 | 54 | 51 | 29 |

**TABLEAU 5 Composition en nombre de résidus du RG II au cours d'une cinétique de dégradation par Penicillium daleae**

| | Initial | 120 h | 168 h | 240 | 264 h | 336 h | 384 h |
|---|---|---|---|---|---|---|---|
| Rhamnose | 4.0 | 3.2 | 3.0 | 2.6 | 2.1 | 0.7 | 0.2 |
| 2-*O*-CH3-Fucose | 1.0 | 1.0 | 1.0 | 0.8 | 0.8 | 0.2 | 0.1 |
| Fucose | 1.0 | 1.0 | 1.0 | 0.7 | 1.0 | 0.5 | 0.6 |
| 2-*O*-CH₃-Xylose | 1.0 | 1.0 | 1.0 | 0.5 | 0.3 | | |
| Apiose | 2.0 | 2.0 | 1.9 | 1.7 | 1.5 | 0.4 | 0.2 |
| Arabinose | 3.0 | 2.6 | 2.2 | 1.8 | 1.4 | 0.4 | 0.2 |
| Galactose | 2.0 | 1.8 | 13 | 1.1 | 0.9 | 0.2 | 0.2 |
| Acide Galacturonique | 10.5 | 10.5 | 7.9 | 7.5 | 7.7 | 4.1 | 4.0 |
| Acide Glucuronique | 1.0 | 1.4 | 0.8 | 0.3 | 0.3 | | |
| Acide Acétique | 0.6 | 0.6 | 0.6 | 0.6 | 0.6 | 0.3 | 0.2 |
| Kdo | 0.9 | 1.9 | 0.9 | 0.5 | 0.9 | 0.7 | 0.6 |
| Dha | 0.7 | 0.11 | 0.3 | 0.3 | 0.6 | 0.6 | 0.5 |
| Total | 27.7 | 27.0 | 21.9 | 18.3 | 18.1 | 8.1 | 6.8 |
| dp moyen de la chaîne | | | | | | | |
| homogalacturonique | 8.5 | 8.0 | 8.0 | 7.5 | 6.0 | 4.5 | 3.5 |
| % molécule résiduelle | 100 | 97 | 79 | 66 | 65 | 29 | 25 |

**Tableau 6 Analyse de méthylation et liaisons des résidus constituant la molécule de RG-II au cours de sa dégradation par P. simplicissimum.**

| Ether Méthylique | Type de liaison | Résidu | Initial | 96 h | 132 h | 160 h | 192 h | 400 h |
|---|---|---|---|---|---|---|---|---|
| 2,3,4-Rha ^{a} | Rha*p* (1→ ^{b} | D2 B6 | 1,3 | 1,45 | 1,0 | 1,1 | 0,7 | 0,2 |
| 3,4-Rha | →2)-Rha*p*-(1→ | B6 | 0,8 | 0,5 | 0,5 | 0,8 | 0,8 | 0,15 |
| 2,4-Rha | →3)-Rha*p*-(1→ | B2 | 1,0 | 1,0 | 1,0 | 1,0 | 1,0 | 1,0 |
| Rha | →2,3,4)-Rha*p*-(1→ | A2 | 1,0 | 0,85 | 0,7 | 0,1 | 0 | 0 |
| 2,3,4-Fuc | 2-*O*-Me-Fuc*p*-(1→ | B4' | 0,7 | 0,7 | 0,7 | 0,7 | 0,6 | 0,6 |
| 2-Fuc | →3,4)-Fuc*p*-(1→ | A3 | 0,6 | 0,6 | 0,4 | 0,1 | 0 | 0 |
| 2,3-Api | →3')-Api-(1→ | B1 A1 | 2,0 | 2,0 | 2,0 | 1,95 | 1,7 | 0,8 |
| 2,3,5-Ara | Ara*f*-(1→ ^{b} | C2 B7 | 1,2 | 1,4 | 1,0 | 1,0 | 0,8 | 0 |
| 2,3,4-Ara | Ara*p*-(1→ | B5 | 0,1 | 0,2 | 0,2 | 0,1 | 0,15 | 0,3 |
| 3,4-Ara | →2)-Ara*p*-(1→ | B5 | 0,2 | 0,4 | 0,55 | 0.7 | 0,7 | 0,4 |
| 4-Ara | →2,3)-Ara*p*-(1→ | B5 | 0,7 | 0,5 | 0,3 | 0,1 | 0 | 0 |
| 2,3,4-Xyl | 2-*0*-Me-Xyl*p*-(1→ | A3' | 0,6 | 0,6 | 0.4 | 0,1 | 0 | 0 |
| 2,3,4,6-Gal | Gal*p*-(1→ | A5 | 0,5 | 0,45 | 0,1 | 0,05 | 0 | 0 |
| 3,6-Gal | →2,4)-Gal*p*-(1→ | B4 | 1,1 | 1,0 | 1,0 | 1,0 | 1,0 | 0,95 |
| 2,6-Gal | →3,4)-Gal*p*-(1→ | A5 | 0,45 | 0,4 | 0,3 | 0,08 | 0 | 0 |

| | | | | | | | | |
|---|---|---|---|---|---|---|---|---|
| Les résultats sont donnés en rapports molaires calculés sur la base de 1 résidu de →3)-Rha*p*-(1→, résidu n° B2 dans la molécule de RG-II et qui reste constant au cours de la dégrac | | | | | | | | |
| ^{a} 2,3,4-Me₃-Rha = 1,5-di-O-acetyl-2,3,4-tri-O-methyl-rhamnitol, etc... | | | | | | | | |
| ^{b} *p* = pyranose, *f* = furanose. | | | | | | | | |

**Tableau 7 Analyse de méthylation et liaisons des résidus constituant le RG-II au cours d'une cinétique de dégradation par Penicillium dalene**

| Ether méthylique | Liaison | Initial | 72 h | 120 h | 168 h | 240 h | 264 h |
|---|---|---|---|---|---|---|---|
| 2,3,4-Rha | Rha*p*→ | 1.4 | 1.2 | 1 | 1.2 | 1.2 | 1.2 |
| 3,4-Rha | →2-Rha*p*→ | 1.1 | 0.7 | 0.5 | 0.4 | 0.3 | 0.4 |
| 2,4-Rha | →3-Rha*p*→ | 1 | 1 | 1 | 1 | 1 | 1 |
| 3-Rha | →2,4-Rha*p*→ | 0.4 | 0.1 | 0.3 | | | |
| Rha | →2,3,4-Rha*p*→ | 1.2 | 1.2 | 1.2 | 1.1 | 0.8 | 0.3 |
| 2,3,4-Fuc | 2-*O*-CH₃-Fuc→ | 0.8 | 0.7 | 0.7 | 0.8 | 0.7 | 0.8 |
| 2-Fuc | →2,4-Fuc→ | 0.6 | 0.7 | 0.7 | 0.6 | 0.3 | 0.2 |
| 2,3,4-Xyl | 2-*O*-CH₃-Xyl→ | 0.6 | 0.6 | 0.5 | 0.5 | 0.3 | 0.1 |
| 2,3-Api | →3'-Api→ | 2.2 | 2.2 | 2.3 | 2.2 | 2.2 | 1.8 |
| 2,3,5-Ara | Ara*f*→ | 1.6 | 1.4 | 1.1 | 1.3 | 0.8 | 0.8 |
| 3,4-Ara | →2-Ara*p*→ | 0.3 | 0.3 | 0.3 | 0.3 | 0.5 | |
| 4-Ara | →2,3-Ara*p*→ | 0.6 | 0.7 | 0.6 | 0.6 | 0.4 | 0.2 |
| 2,3,4,6-Gal | Gal*p*→ | 0.5 | 0.5 | 0.5 | 0.4 | 0.3 | 0.2 |
| 2,6-Gal | →3,4-Gal*p*→ | 0.6 | 0.6 | 0.5 | 0.5 | 0.4 | 0.3 |
| 3,6-Gal | →2,4-Gal*p*→ | 1.0 | 1.2 | 1.1 | 1.1 | 1.0 | 0.9 |
| 3-Acer | →2-AccrA→ | 0.5 | 0.5 | 0.3 | 0.5 | 0.6 | 0.6 |
| 2,3,4-GalA | Gal*p*A→ | 2.6 | 3.0 | 3.0 | 2.6 | 1.9 | 1.4 |
| 2,3-GalA | →4-Gal*p*A→ | 2.0 | 2.3 | 2.1 | 1.8 | 1.7 | 1.5 |
| 2-GalA | →3,4-Gal*p*A→ | 1.5 | 1.5 | 1.5 | 1.5 | 1.3 | 1.3 |
| 3-GalA | →2,4-Gal*p*A→ | 1.1 | 1.2 | 1.3 | 1.3 | 1.8 | 1.6 |
| GalA | →2,3,4-Gal*p*A→ | 0.8 | 0.9 | 0.8 | 0.7 | 0.3 | 0.2 |
| 1,2,3,5-GalA | →4-Gal*p*A réduit | 1.0 | 0.9 | 0.8 | 0.7 | 0.7 | 0.8 |
| 2,3,4-GlcA | Gl*cp*A→ | | 0.2 | | | | |
| 3,4GlcA | →2-Gl*cp*A→ | 1.1 | 1.4 | 1.3 | 1.1 | 0.6 | 0.4 |

Les résultats sont donnés sur la base de 1 résidu de →3-Rha*p*→, résidu B2 dans la molécule de RG-II qui reste constant au cours de la dégradation
2,3,4-Rha = 1,5-di-O-acétyl-2,3,4-tri-O-méthyl-rhamnitol, etc...

## Revendications

1. Préparation enzyzmatique susceptible d'être obtenue à partir d'un champignon du genre Penicillium, **caractérisée en ce qu'**elle présente une activité de dégradation du rhamnogalacturonane II (RG-II), **en ce qu'**elle présente une activité endo-β-L-rhamnopyranosyl-(1→3')-D-apiofuranosyl hydrolase et **en ce qu'**elle présente une activité endo-α-L-fucopyranosyl-(1→4)-L-rhamnopyranosyl hydrolase.

2. Souche de champignon produisant une enzyme selon la revendication 1, **caractérisée en ce qu'**elle est déposée auprès de la CNCM sous le n°I-1578 (LAV2).

3. Souche de champignon produisant une enzyme selon la revendication 1, **caractérisée en ce qu'**elle est déposée auprès de la CNCM sous le n°I-1577 (IPV1).

4. Procédé d'obtention d'une préparation selon la revendication 1 comprenant les étapes suivantes :
- mise en culture de microorganismes Penicilium simplicissimum ou Penicilium daleae dans un milieu de culture contenant du R6II ou l'un de ses dérivés,
- récupération de la préparation enzymatique dans le surnageant du broyat des microorganismes.

5. Procédé selon la revendication 4 **caractérisé en ce qu'**il comprend les étapes suivantes:
- mise en culture de microorganismes Penicillium simplicissimum ou Penicillium daleae dans un milieu de culture adapté aux microorganismes contenant du RG-II, ou l'un de ses dérivés,
- récupération des microorganismes,
- broyage des microorganismes,
- élimination du matériel insoluble, et
- récupération du surnageant contenant l'enzyme ou la préparation enzymatique.

6. Utilisation d'une préparation enzymatique selon la revendication 1 pour la dégradation ou la modification du RG-II.

7. Utilisation d'une préparation enzymatique selon la revendication 1 pour améliorer la filtrabilité et faciliter la préparation de jus concentrés, ou pour favoriser la clarification.

8. Utilisation d'une préparation enzymatique selon la revendication 1 pour le nettoyage des supports de filtration utilisés pour la filtration des jus de fruits, de légumes et de leurs dérivés.

9. Utilisation d'une préparation enzymatique selon la revendication 1 pour la macération, la liquéfaction ou l'hydrolyse totale ou partielle de tissus végétaux.

10. Utilisation selon la revendication 10 dans les préparations de type macérase pour la production de nectars de fruits, de purées, de gelées et de concentrés de fruits, de légumes et de leurs dérivés, y compris le vin.

11. Utilisation selon la revendication 10 dans les préparations liquéfiantes pour la production de jus et de bases aromatiques de fruits, de légumes et de leurs dérivés, y compris le vin, et la production de bière.

12. Utilisation selon la revendication 10 pour la production de pectines à partir de résidus de végétaux tels que des pulpes de betterave et des résidus solides après pressurage des fruits.

13. Utilisation selon la revendication 10 pour la production d'aliments pour animaux à partir de matériel végétal.

14. Utilisation selon la revendication 10 pour la production de cellulose à partir de végétaux pour l'industrie textile, en particulier à partir de coton, et pour la production de papier.

15. Utilisation selon la revendication 10 pour la production à partir de résidus de végétaux, d'oligosaccharides à activité élicitrice de réactions de défense des plantes et pouvant être utilisées comme produits phytosanitaires.

## Claims

1. Enzymatic preparation liable to be obtained from a fungus of Penicillium genus, **characterised in that** it has an activity for degrading rhamnogalacturonane II (RG-11), an endo-β-L-rhamnopyranosyl-(1→3')-D-apiofuranosyl hydrolase activity and an endo-α-L-fucopyranosyl-(1→4)-L-rhamnopyranosyl hydrolase activity.

2. Fungus strain giving an enzyme according to claim 1, **characterised in that** the it is filed at the CNCM under n° 1-1578 (LAV2).

3. Fungus strain giving a enzyme according to claim 1, **characterised in that** it is filed at the CNCM under n° 1-1577 (IPV1).

4. Method for obtaining a preparation according to claim 1, comprising the following steps:
- cultivating *Penicillium simplicissimum* or *Penicillium daleae* microorganisms in a culture medium comprising R6II or a derivative thereof,
- recovery of the enzymatic preparation in the supernatant from the crushed microorganisms.

5. Method according to claim 4, **characterised in that** it comprises the following steps:
- cultivating *Penicillium simplicissimum* or *Penicillium daleae* microorganisms in a culture medium adapted to the microorganisms comprising RG-II or a derivative thereof ;
- recovery of the microorganisms,
- crushing of the microorganisms,
- elimination of the non soluble materials, and
- recovery of the supernatant containing the enzyme or the enzymatic preparation.

6. Use of an enzymatic preparation according to claim 1 for degrading or modifying RG-II.

7. Use of an enzymatic preparation according to claim 1 for improving filtering capacity and facilitate the preparation of concentrated juices or improve clarification.

8. Use of an enzymatic preparation according to claim 1 for cleaning the filtering supports used for filtering the juices of fruits, vegetables and derivatives thereof.

9. Use of an enzymatic preparation according to claim 1 for the maceration, liquefaction or full or partial hydrolysis of plant tissues.

10. Use according to claim 9, in preparations of the macerase type, for the production of fruit nectars, purees, jellies, and of fruit and vegetable concentrates and their derivatives, including wine.

11. Use according to claim 10, in liquifying preparations for the production of juices and aromatic bases from fruits, vegetables, and their derivatives, including wine, and for the production of beer.

12. Use according to claim 10, for the production of pectins from residues of plants such as beetroot pulp and solid residues from the squeezing of fruits.

13. Use according to claim 10, for the production of animal food from vegetal material.

14. Use according to claim 10, for the production of cellulose from plants for the textile industry, in particular from cotton, and for the production of paper.

15. Use according to claim 10, for the production from plan residues of oligosaccharides having enhancing activities of the protecting reactions of plants and which can be used as phytosanitary products.

## Patentansprüche

1. Enzympräparat, das ausgehend von einem Pilz der Gattung *Penicillium* erhalten werden kann, **dadurch gekennzeichnet, dass** es eine Aktivität bezüglich des Abbaus von Rhamnogalacturonan II (RG-II) aufweist, dass es eine endo-β-L-Rhamnopyranosyl-(1→3')-D-apiofuranosyl-Hydrolase-Aktivität aufweist und dass es eine endo-α-L-Fucopyranosyl-(1→4)-L-rhamnopyranosyl-Hydrolase-Aktivität aufweist.

2. Pilzstamm, der ein Enzym gemäß Anspruch 1 erzeugt, **dadurch gekennzeichnet, dass** er beim CNCM unter der Nr. 1-1578 (LAV2) hinterlegt ist.

3. Pilzstamm, der ein Enzym gemäß Anspruch 1 erzeugt, **dadurch gekennzeichnet, dass** er beim CNCM unter der Nr. 1-1577 (IPV1) hinterlegt ist.

4. Verfahren zur Gewinnung eines Präparats gemäß Anspruch 1, das die folgenden Schritte umfasst:
- Kultivieren von Mikroorganismen der Art *Penicillium simplicissimum* oder *Penicillium daleae* in einem Kulturmedium, das RGII oder eines seiner Derivate enthält;
- Gewinnung des Enzympräparats aus dem Überstand des Homogenisats der Mikroorganismen.

5. Verfahren gemäß Anspruch 4, **dadurch gekennzeichnet, dass** es die folgenden Schritte umfasst:
- Kultivieren von Mikroorganismen der Art *Penicillium simplicissimum* oder *Penicillium daleae* in einem an die Mikroorganismen angepassten Kulturmedium, das RGII oder eines seiner Derivate enthält;
- Gewinnung der Mikroorganismen;
- Homogenisieren der Mikroorganismen;
- Entfernung des unlöslichen Materials; und
- Gewinnung des Überstands, der das Enzym oder das Enzympräparat enthält.

6. Verwendung eines Enzympräparats gemäß Anspruch 1 zum Abbau oder zur Modifikation von RG-II.

7. Verwendung eines Enzympräparats gemäß Anspruch 1 zur Verbesserung der Filtrierbarkeit und Erleichterung der Herstellung von konzentrierten Säften oder zur Förderung der Klärung.

8. Verwendung eines Enzympräparats gemäß Anspruch 1 zur Reinigung der Filtrationsträger, die zur Filtration von Fruchtsäften, Gemüsen und ihren Derivaten verwendet wurden.

9. Verwendung eines Enzympräparats gemäß Anspruch 1 zur Mazeration, Verflüssigung oder vollständigen oder partiellen Hydrolyse von Pflanzengeweben.

10. Verwendung gemäß Anspruch 10 in Präparaten des Typs Macerase zur Herstellung von Fruchtnektaren, -musen, -gelees und -konzentraten, Gemüsen und ihren Derivaten einschließlich Wein.

11. Verwendung gemäß Anspruch 10 in Verflüssigungspräparaten zur Herstellung von Saft und aromatischen Fruchtbasen, Gemüsen und ihren Derivaten einschließlich Wein und die Herstellung von Bier.

12. Verwendung gemäß Anspruch 10 zur Herstellung von Pectinen aus Pflanzenrückständen, wie Rübenschnitzel und festen Rückständen vom Pressen der Früchte.

13. Verwendung gemäß Anspruch 10 zur Herstellung von Nahrungsmitteln für Tiere aus pflanzlichem Material.

14. Verwendung gemäß Anspruch 10 zur Herstellung von Cellulose aus Pflanzen für die Textilindustrie, insbesondere aus Baumwolle, und zur Herstellung von Papier.

15. Verwendung gemäß Anspruch 10 zur Herstellung von Oligosacchariden mit Elicitor-Aktivität bezüglich Abwehrreaktionen von Pflanzen, die als Pflanzenschutzmittel verwendet werden können, aus Pflanzenrückständen.
